(19) Europäisches Patentamt
European Patent Office
Office européen des brevets

(11) EP 1 516 045 B1

(12) **EUROPÄISCHE PATENTSCHRIFT**

(45) Veröffentlichungstag und Bekanntmachung des Hinweises auf die Patenterteilung:
**25.02.2009 Patentblatt 2009/09**

(21) Anmeldenummer: **03759889.3**

(22) Anmeldetag: **09.05.2003**

(51) Int Cl.:
**C12N 5/00** (2006.01)

(86) Internationale Anmeldenummer:
**PCT/EP2003/004875**

(87) Internationale Veröffentlichungsnummer:
**WO 2003/106661 (24.12.2003 Gazette 2003/52)**

(54) **VERWENDUNG VON GLUTAMINFREIEN MEDIUM**

USE OF GLUTAMIN-FREE MEDIUM

UTILISATION DE MILIEU EXEMPT DE GLUTAMINE

(84) Benannte Vertragsstaaten:
**AT BE BG CH CY CZ DE DK EE ES FI FR GB GR HU IE IT LI LU MC NL PT RO SE SI SK TR**

(30) Priorität: **13.06.2002 DE 10226455**

(43) Veröffentlichungstag der Anmeldung:
**23.03.2005 Patentblatt 2005/12**

(73) Patentinhaber: **Alt, Rüdiger**
**04155 Leipzig (DE)**

(72) Erfinder: **Alt, Rüdiger**
**04155 Leipzig (DE)**

(74) Vertreter: **Schrell, Andreas**
**Gleiss Grosse Schrell & Partner**
**Patentanwälte Rechtsanwälte**
**Leitzstrasse 45**
**70469 Stuttgart (DE)**

(56) Entgegenhaltungen:
**US-A- 4 049 494**

- **DOWNS STEPHEN M ET AL: "Energy substrates and the completion of spontaneous meiotic maturation" ZYGOTE, Bd. 8, Nr. 4, November 2000 (2000-11), Seiten 339-351, XP008025824 ISSN: 0967-1994**
- **DATABASE WPI Section Ch, Week 199902 Derwent Publications Ltd., London, GB; Class B04, AN 1999-022688 XP002265010 QUEST INT: "Use of protein hydrolysates in cell culture media - as replacements for serum, glutamine and other free amino acids" -& RESEARCH DISCLOSURE, Bd. 415, Nr. 051, 10. November 1998 (1998-11-10), Seiten 1-3, XP007123516 Emsworth, GB**
- **NEERMANN J ET AL: "COMPARATIVE ANALYSIS OF GLUCOSE AND GLUTAMINE METABOLISM IN TRANSFORMED MAMMALIAN CELL LINES, INSECT AND PRIMARY LIVER CELLS" JOURNAL OF CELLULAR PHYSIOLOGY, LISS, NEW YORK, NY, US, Bd. 166, Nr. 1, 1996, Seiten 152-169, XP000929952 ISSN: 0021-9541 in der Anmeldung erwähnt**
- **SCHNEIDER M ET AL: "The importance of ammonia in mammalian cell culture" JOURNAL OF BIOTECHNOLOGY, Bd. 46, Nr. 3, 15. Mai 1996 (1996-05-15), Seiten 161-185,**

**EP 1 516 045 B1**

**Beschreibung**

Die vorliegende Erfindung betrifft die Verwendung eines Zellkulturmediums, für die Kultivierung von Säugerzellen, Verfahren zur Kultivierung von Säugetierzellen, wobei die Bildung von Lactat und/oder Ammonium in einer Zellkultur vermindert wird.

Der Stand der Technik in der Kultivierung von insbesondere Säugetierzellen für biotechnologische Anwendungen sieht vor, dass Zellkulturmedien eingesetzt werden, welche unter anderem Glutamin, das heißt insbesondere L-Glutamin, und/oder glutaminhaltige Stoffe enthalten. Insbesondere für die industrielle Produktion müssen geeignete Wachstumsbedingungen für die Teilung und Vermehrung von tierischen Zellen, insbesondere Säugerzellen, geschaffen werden. Üblicherweise werden tierische Zellen in geeigneten Bioreaktoren kultiviert. Mit Hilfe der Zellkulturtechnik können so Produktionssysteme aus der Kultur tierischer Zellen entwickelt werden, die immer mehr zu einer Alternative zu bakteriellen Proteinexpressionssystemen heranwachsen und das Spektrum produzierbarer, insbesondere tierischer Proteine erweitern. Besonders bei komplexen Proteinen, die nach der Proteinsynthese prozessiert oder glycosyliert werden müssen, oder bei der Herstellung von Antikörpern ist die Verwendung von Säugetierzellen oder Hybridomazellen Mittel der Wahl. Auch zur Herstellung von Viren, insbesondere als Impfstoffe, gibt es in den meisten Fällen keine Alternative zu einer geeigneten tierischen oder menschlichen Wirtszelle.

In solchen Zellkultursystemen werden meist kontinuierlich wachsende Zellen, welche entweder genetisch modifizierte Zellen oder Tumorzellen sind, eingesetzt. Daneben existieren Kulturen aus primären Zellen, welche direkt aus Geweben von Tieren oder Menschen entnommen werden, sich aber meist nur wenige Male teilen können.

Um das Wachstum der Zellen in Kultur zu fördern, verwendet man spezielle Zellkulturmedien und Apparate, die geeignete Bedingungen für das Zellwachstum schaffen. Von besonderer Bedeutung für ein kontinuierliches Zellwachstum sind unter anderem die Sterilität der Kultur, die Parameter Temperatur, pH-Wert, Osmolarität und Sauerstoffpartialdruck sowie das Nährstoffangebot, insbesondere in Form von Kohlenstoffquellen und Stickstoffquellen, beispielsweise Zucker und/oder Aminosäuren. Als Substrate des Energiestoffwechsels der Zellen stehen in klassischen bekannten Zellkulturmedien Kohlenhydrate, meist Glucose, und L-Aminosäuren, hauptsächlich Glutamin, zur Verfügung. Glucose und Glutamin sind sowohl als Kohlenstoffquellen wie auch für die Energiegewinnung wichtig. Glutamin ist zudem die Hauptstickstoffquelle in klassischen Zellkulturmedien.

Glucose wird zu einem großen Teil von den kultivierten Zellen anaerob zu Lactat umgesetzt. Lactat bewirkt dabei eine Ansäuerung des Mediums. Im Verlauf der Kultivierung sinkt daher der pH-Wert im Zellkulturmedium. Lactat ist also eines der typischen Endprodukte welche aus den kultivierten Zellen in das Medium abgegeben werden. Ein direkter Zusammenhang zwischen Glucoseaufnahme und Lactatbildung ist bekannt (zum Beispiel Fitzpatrick et al., Appl. Biochem. Biotechnol. (1993) 43/2:93-116); Neermann und Wagner, J. Cell. Physiol. (1996) 166:152-169). Für Hybridomazellen sowie weitere immortalisierte Säugetierzellen wie die Baby Hamster Kidney Zelllinie (BHK) oder die Chinese Hamster Ovary Zelllinie (CHO) konnte gezeigt werden, dass Glucose die Hauptquelle für die Lactatakkumulation im Zellkulturmedium ist. Der Flux von Glucose zu Lactat durch die Glycolyse ist in allen kontinuierlichen Säugerzellen sehr hoch. Demgegenüber ist der Übergang von der Glycolyse in den Zitratzyklus gering.

Lactat hat in hohen Konzentrationen negative Einflüsse auf Zellwachstum und Produktbildung und wirkt somit als Hemmstoff. Doch konnte gezeigt werden, dass Lactatkonzentrationen im Medium von 20 mmol/l und mehr die Produktbildung hemmen. Bei Lactatkonzentrationen von mehr als 60 mmol/l kommt es zu einer signifikanten Hemmung des Zellwachstums (Glacken, Bio/Technology (1988) 6:1941-1950). Eine Reduzierung der Lactatakkumulation in den Zellkulturmedien wird daher angestrebt.

Es zeigt sich, dass der Energiebedarf, der nicht durch Glucose gedeckt wird, je nach Zelllinie durchschnittlich 30 bis 98 %, durch die Verstoffwechselung der Aminosäure Glutamin gedeckt wird. Aus Reitzer et al. (J. Biol. Chem. 254/8 (1979):2669-2676) ist bereits seit den 70er Jahren bekannt, dass beispielsweise für die immortalisierte Zelllinie des Zervixkarzinoms von Helene Lachs (HeLa-Zellen) Glutamin die Hauptenergiequelle darstellt. Glutamin spielt für die schädliche Lactatbildung im Medium eine nur untergeordnete Rolle, es ist jedoch hauptverantwortlich für die Akkumulation von Ammoniumionen in den Zellkulturmedien. Ammonium entsteht aus Glutamin bei einer Reaktion erster Ordnung, bei der die Aminogruppe des Glutamins mit der eigenen Aminosäuregruppe reagiert und unter Abspaltung eines Moleküls Ammonium ($NH_4^+$) ein zyklisches Molekül Pyrrolydoncarboxylat bildet. Diese Zersetzungsreaktion ist temperaturabhängig und findet in allen glutaminhaltigen Medien statt. Die Instabilität des Glutamins kann unter Kulturbedingungen und bei längeren Lagerzeiten zu einer Ansammlung beträchtlicher Ammoniummengen in den bekannten Kulturmedien führen. Ammonium entsteht auch über die Glutaminolyse im Stoffwechsel der kultivierten Zellen. In der Glutaminasereaktion (Umwandlung von Glutamin in Glutamat) wird aus jedem umgesetzten Glutaminmolekül ein Ammoniummolekül frei. Praktisch wird dabei aus jedem von der Zelle aufgenommenen Glutaminmolekül ein Ammoniummolekül in das Kulturmedium freigesetzt. Bei der üblichen Glutaminkonzentration in bekannten Zellkulturmedien, welche zwischen 2 und 8 mmol/l liegt, entstehen als Abbauprodukt des Glutamins entsprechende Mengen Ammonium im Zellkulturmedium.

Problematisch ist, dass Ammonium als Hemmstoff beziehungsweise als Zellgift wirkt. Bereits nach wenigen Tagen Kultivierung in glutaminhaltigen Zellkulturmedien ergeben sich negative Effekte auf die Produktbildung und das Wachstum der Zellen im Medium aufgrund der Ammoniumakkumulation im Medium. Ammonium hat einen, je nach eingesetzter Zelllinie auch außerordentlichen, Einfluss auf die Zellteilungsrate. Gleichzeitig wird in vielen Fällen vermehrter Zelltod in der Zellkultur festgestellt. Darüber hinaus hat Ammonium einen unmittelbaren Einfluss auf den Zellmetabolismus und auf den Energiemetabolismus der kultivierten Zellen. Dies wirkt sich vor allem, entweder direkt oder indirekt, auf die Produktbildungsrate sowie auf die Qualität der gebildeten Produkte aus (Ozturk, In: Encyclopedia of Cell Technology (Hrsg. E. Spier) John Wiley & Sons, New York, 2000:121-136). Ammonium hat dabei Einfluss auf die Energieladung und auf das Nucleotidverhältnis beziehungsweise die intrazellulären Nucleotidkonzentrationen der Zellen. Bei hohen Ammoniumkonzentrationen beobachtet man veränderte Glycosylierungsmuster an Glycoproteinen und verringerte Ausbeuten bei heterolog exprimierten Proteinen, darunter auch wichtige therapeutische Proteine und Antikörper (Schneider et al., J. Biotechnol. (1996) 46:161-185). Eine Reduzierung der Ammoniumakkumulation in den Zellkulturmedien wird daher angestrebt.

Verfahren zur Reduzierung der Lactat- und/oder Ammoniumkonzentration in Zellkulturmedien sind bekannt. Ein Verfahren besteht im regelmäßigen Mediumswechsel beispielsweise über Perfusionskultursysteme. Dieses Verfahren ist apparativ meist aufwendig und erfordert zudem einen hohen Mediumsverbrauch. Gleichzeitig verbietet dieses Verfahren aufgrund der häufigen Medientausche auch die Akkumulation gewünschter Produkte im Medium, so dass zur Gewinnung dieser Produkte beispielsweise aufwendige Aufkonzentrierungs- und Aufreinigungsverfahren durchgeführt werden müssen. Ein weiteres Verfahren besteht in der selektiven Entfernung von Ammonium und/oder Lactat beispielsweise durch Membransysteme, insbesondere in Form der Dialyse. Akkumuliertes Ammonium kann auch durch spezifische Ammoniumadsorber, Ionenaustauscher oder Elektrodialysegeräte entfernt werden. Diese Verfahren zeichnen sich ebenfalls durch einen hohen apparativen Aufwand aus und sind außerdem kostenintensiv.

Andere Strategien zur Ammoniumentfernung bestehen darin, in den eingesetzten Zellkulturmedien Glutamin durch Glutamin-Ersatzstoffe wie Glutamat, Asparagin oder α-Ketoglutarat zu ersetzen (zum Beispiel Kurano et al., J. Biotechnol. (1990) 15:113-128). Entscheidend ist dabei jedoch, ob die kultivierten Zellen überhaupt in der Lage sind, solche Glutamin-Ersatzstoffe zu verwerten, da heißt zum Beispiel Glutamat an Stelle von Glutamin aufzunehmen und zu verstoffwechseln. Es wurde gezeigt, dass Zellen der Zelllinie Madin Darby Canine Kidney (MDCK) nicht in glutaminfreiem Medium kultiviert werden können, wenn Glutamin durch Glutamat ersetzt wird (Mc-Dermott und Butler, J. Cell. Sci. (1993) 104:51-58). Aus Altamirano et al. (Animal Cell Technology: Products from Cells, Cells as Products (1999): 95-97) ist ein Beispiel für die Anwendung einer Fed-Batch-Kultur mit CHO-Zellen bekannt, bei der Glutamin durch Glutamat sowie Glucose durch verschiedene Hexosen ersetzt wurden. Trotz reduzierter Ammoniumbildung wurde dabei keine Verbesserung des Wachstumsprofils der kultivierten Zellen erreicht. Ebenso führte die Substitution von L-Glutamin mit L-Asparagin lediglich zu einer 40 %igen Ammoniumreduktion (Schneider et al., a.a.O.). Fed-Batch-Kulturführung mit limitierter Glucose- beziehungsweise Glutaminkonzentration führte jeweils auch zu verringerter Produktbildung (Nadeau et al., Met. Eng. (2000) 2:277-292; Doverskog et al., J. Biotechnol. (1997) 59:103-115). Downs und Hudson (Zyqote (2000) 8: 339-351) beschreiben die Verwandlung eines glutaminfreien Mediums mit bis zu 2,3 mmol/l Pyruvat zur Reifung von Maus-Dozyten. Von Neermann und Wagner (a.a.O.) ist bekannt, dass bei der Kultivierung von CHO-Zellen fast keine Kohlenstoffatome von der Glycolyse über die Pyruvatdehydrogenase in den Zitratzyklus eintreten. Zur Betreibung des Zitratzyklus muss fast allein Glutamin als Kohlenstoffquelle dienen. Daraus wird geschlossen, dass beim Fehlen von Glutamin ein nicht wettzumachender Mangel an Kohlenstoffquellen im Zitratzyklus entsteht. Die Lehrmeinung verbietet also den Verzicht von Glutamin in Zellkulturmedien.

Bekannte Zellkulturmedien, die für die Verwendung als Nährlösung für tierische Zellen, insbesondere Säugerzellen, vorgesehen sind, enthalten daher grundsätzlich Glutamin und/oder glutaminhaltige Verbindungen. Als glutaminhaltige Verbindungen werden insbesondere Oligopeptide wie Dipeptide aus L-Glutamin und L-Alanin wie L-Alanyl-L-Glutamin, welche beispielsweise unter dem Namen Glutamax® vertrieben werden, eingesetzt. Diese glutaminhaltigen Verbindungen degradieren nicht spontan. Dadurch wird zumindest die durch die spontane Degradation von Glutamin verursachte Ammonium-Last im Zellkulturmedium verhindert. Ein bekanntes Medium enthält folgende Hauptbestandteile: 1.) L-Aminosäuremischung, inklusive L-Glutamin und/oder L-glutaminhaltige Verbindung(en), 2.) Vitaminmischung, 3.) Salz und Puffermischung sowie 4.) mindestens eine Kohlenstoffquelle, die vergoren oder anders im Stoffwechsel der Zellen oxidiert wird, beispielsweise Zucker wie Glucose. Tierische Zellen, insbesondere Säugetierzellen, können bestimmte Aminosäuren nicht selbst de novo synthetisieren. Diese essenziellen Aminosäuren sind daher ebenfalls Bestandteile der bekannten Zellkulturmedien. Häufig werden weitere auch nicht-essenzielle Aminosäuren in verschiedenen Konzentrationen in die Zellkulturmedien gegeben, um das Wachstum der Zellen zu beschleunigen. Auch werden undefinierte Proteinhydrolysate wie Pepton zugesetzt. Die Aminosäurezusammensetzungen der meisten bekannten Zellkulturmedien gehen insbesondere auf Arbeiten von Eagle (Science (1955) 122:501; Science (1959) 130: 432-437) und Dulbecco (Virology (1959) 8:396-397) aus den 50er Jahren zurück und bestehen bis heute im Wesentlichen unverändert.

Demgemäß besteht das der vorliegenden Erfindung zugrunde liegende technische Problem im Wesentlichen in der Bereitstellung von Zellkulturmedien für die Kultivierung von Säugetierzellen, Verwendung dieser Zellkulturmedien sowie Verfahren zur Kultivierung von Zellen, wobei die Bildung von Lactat und/oder Ammonium in einer Zellkultur verhindert oder verringert wird und gleichzeitig das Zellwachstum und/oder die Produktbildung der kultivierten Zellen nicht gehemmt wird.

Dieses Problem wird erfindungsgemäß durch ein Verfahren zur Kultivierung von Säugetierzellen mit einem Zellkulturmedium gelöst, welches insbesondere für die Kultivierung von tierischen Zellen, bevorzugt Säugetierzellen, geeignet ist und welches dadurch gekennzeichnet ist, dass es im Wesentlichen frei von Glutamin und/oder glutaminhaltigen Stoffen ist. Erfindungsgemäß weist das Kulturmedium gleichzeitig Pyruvat und/oder mindestens eine pyruvathaltige Verbindung, das heißt mindestens ein pyruvathaltiger Ersatzstoff, in einer Endkonzentration von insbesondere 2, 5 bis 100 mmol/l, bevorzugt von 5 bis 60 mmol/l, besonders bevorzugt 5 bis 20 mmol/l, auf. Dabei dient Pyruvat und/ oder die mindestens eine Pyruvat enthaltende Verbindung als mindestens eine Kohlenstoffquelle im Zellkulturmedium. Selbstverständlich ist vorgesehen, dass in dem erfindungsgemäß verwendeten Zellkulturmedium weitere Kohlenstoffquellen vorhanden sind. Erfindungsgemäß bevorzugt weist das Zellkulturmedium mindestens eine weitere Kohlenstoffquelle auf, welche vergoren oder auf andere Weise im Stoffwechsel der zu kultivierenden Zellen zur Energieerzeugung dient, beispielsweise durch Zellatmung. Es ist auch vorgesehen, dass das erfindungsgemäß verwendete im Wesentlichen glutaminfreie Zellkulturmedium mindestens eine Stickstoffquelle enthält. Besonders bevorzugt dient die mindestens eine Stickstoffquelle und/oder mindestens eines ihrer Abbauprodukte auch als Energiequelle, welche vergoren wird und/oder auf andere Weise, beispielsweise durch Zellatmung, zur Energiegewinnung in den zu kultivierenden Zellen genutzt wird. Selbstverständlich ist vorgesehen, dass das erfindungsgemäß verwendete Kulturmedium bevorzugt weitere, in bekannten Zellkulturmedien vorhandene Inhaltsstoffe wie Aminosäuren, Glutamin ausgenommen, Vitamine, anorganische und/ oder organische Salze, beispielsweise enthaltend die Metallkationen Natrium, Kalium, Magnesium, Kalzium und/oder Eisen sowie anorganische Anionen wie Carbonat, Hydrogencarbonat, Sulfat, Phosphat, Hydrogenphosphat, Chlorid und/oder Nitrat aufweist. Weiter ist vorgesehen, dass das erfindungsgemäß verwendete Zellkulturmedium mindestens ein Puffersystem, beispielsweise aus den vorgenannten anorganischen und organischen Salzen wie Bicarbonatpuffer oder Phosphatpuffer, oder aber aus weiteren organischen und/oder anorganischen Verbindungen, beispielsweise die bekannten Puffersysteme HEPES, TRIS, MOPS, BES, TES, DIPSO, MOBS, TAPSO oder vergleichbare aufweist.

Erfindungsgemäß ist bevorzugt vorgesehen, dass das Zellkulturmedium frei von Proteinen, Peptiden und/oder Serum ist. In einer alternativen bevorzugten Ausführungsform enthält das Zellkulturmedium auch mindestens ein Protein und/oder Proteingemisch und/oder eine verdaute und/oder hydrolysierte Verarbeitungsform von Proteinen oder proteinreichen Ausgangsstoffen, wie Hefeextrakte oder Proteinhydrolysate, beispielsweise aus Soja, Milch oder Fleisch, insbesondere in Form von löslichen Di-, Tri-, und Polypeptiden, beispielsweise Pepton (z.B. Difco, Inc., USA) wie Sojapepton, Caseinpepton oder Fleischpepton, und/oder Peptidgemische aufweist. Bevorzugt werden 0,2 % Pepton (Endkonzentration) im erfindungsgemäßen Medium eingesetzt. Erfindungsgemäß bevorzugt weist das Zellkulturmedium auch Anteile von mindestens einem Blutserum und/oder mindestens einen Bestandteil von Blutseren auf. Diese Blutseren oder Blutserumsbestandteile stammen bevorzugt aus Säugetierorganismen wie Schwein, Rind, Pferd, Kaninchen, Ziege und Mensch.

Bevorzugt werden 5 % bis 10 % Serum eingesetzt. Insbesondere wird fötales Kälberserum (FCS) eingesetzt. Soll das erfindungsgemäß verwendete Medium frei von tierischen Bestandteilen sein, muss insbesondere auf den Zusatz von Seren und Serumbestandteilen verzichtet werden; eine Kontamination von Seren mit Viren und/oder Prionen kann nicht ausgeschlossen werden.

In einer besonders bevorzugten Ausführungsform des erfindungsgemäß verwendeten Kulturmediums ist die Grundform dieses Mediums aus üblichen Standardzusammensetzungen zur Herstellung von Zellkulturmedien abgeleitet mit der Maßgabe, dass Glutamin und/oder glutaminhaltige Stoffe in diesen Zusammensetzungen fehlen. Formulierungen bekannter Standardzusammensetzungen gehen im Wesentlichen auf Rezepte von Eagle (a.a.O.) und Dulbecco (a.a.O.) zurück oder sind Variationen dieser Rezepte. Erfindungsgemäß bevorzugt sind als Standardzusammensetzungen "Glasgow modified Eagle's medium / Glasgow minimum essential medium" (GMEM), BHK-12 Medium, "Dulbecco's modified Eagle's medium" (DMEM), "Basal Eagle's medium" (BME), "Minimum essential medium" (MEM) oder "Iscove's modified Dulbecco's medium" (IMDM). In einzelnen ausgewählten Standardzusammensetzungen können teilweise schon Anteile an Pyruvat oder pyruvathaltigen Verbindungen, üblicherweise bis maximal 1 mmol/l, vorhanden sein. Selbstverständlich ist auch vorgesehen, dass in dem erfindungsgemäßen Verfahren weitere Inhaltsstoffe in Zellkulturmedien eingesetzt werden, die das Wachstum von Zellen fördern.

Im Zusammenhang mit der vorliegenden Erfindung wird unter der Formulierung "im Wesentlichen glutaminfrei" oder ähnlichen Formulierungen verstanden, dass der Anteil an Glutamin, insbesondere L-Glutamin und glutaminhaltigen Verbindungen, welche einer Medienzusammensetzung zugeführt wurden, Null ist. Selbstverständlich können geringe Anteile von Glutamin oder glutaminhaltigen Verbindungen, welche in anderen Komponenten der Medienzusammensetzung unvermeidlich, beispielsweise als Verunreinigung, enthalten sind, im Medium vorhanden sein. Insbesondere beträgt daher der Anteil an Glutamin oder glutaminhaltiger Verbindungen im Medium stets weniger als 1 mmol/l, oder weniger als 0,5 mmol/l, erfindungsgemäß weniger als 0,2 mmol/l, weniger als 0,1 mmol/l oder weniger als 0,05 mmol/l. Erfindungsgemäß besonders bevorzugt sind Medien, die völlig frei von Glutamin und glutaminhaltigen Verbindungen sind.

Beschrieben ist auch ein Verfahren zur Herstellung eines im Wesentlichen glutaminfreien, insbesondere glutaminfreien, Zellkulturmediums, umfassend im Wesentlichen die Schritte:

a) Zugabe von mindestens einer Stickstoffquelle mit der Maßgabe, dass als die mindestens eine Stickstoffquelle kein Glutamin und/oder keine glutaminhaltigen Stoffe eingesetzt werden,

b) Zugabe von mindestens einer Kohlenstoffquelle mit der Maßgabe, dass als die mindestens eine Kohlenstoffquelle kein Glutamin und/oder keine glutaminhaltigen Stoffe eingesetzt werden und dass als die mindestens eine Kohlenstoffquelle Pyruvat und/oder mindestens ein pyruvathaltiger Ersatzstoff eingesetzt wird,

wobei bevorzugt mindestens ein Pyruvat-Salz in einer Endkonzentration insbesondere von 2 bis 100 mmol/l, vorzugsweise von 5 bis 60 mmol/l, besonders bevorzugt von 5 bis 20 mmol/l, eingesetzt wird.

Erfindungsgemäß bevorzugt wird als Pyruvat Natriumsalz eingesetzt. Es ist aber auch vorgesehen, an Stelle des Pyruvats und/oder zusätzlich zu dem mindestens einen Pyruvat, die freie Säure des Pyruvats, Brenztraubensäure, einzusetzen.

Ein weiterer Gegenstand der Erfindung ist auch ein Verfahren zur Kultivierung von Säugetierzellen, welches dadurch gekennzeichnet ist, dass das vorgenannte Zellkulturmedium beziehungsweise das mit dem vorgenannten Verfahren erhaltene Zellkulturmedium eingesetzt wird. Erfindungsgemäß ist ein Verfahren zur Kultivierung von Zellen, Geweben oder Organen von Säugetieren oder Menschen unter Verwendung eines Mediums, welches dadurch gekennzeichnet ist, dass

a) die Konzentration von L-Glutamin und/oder glutaminhaltiger Ersatzstoffe für L-Glutamin, beispielsweise glutaminhaltige Oligopeptide wie L-Alanyl-L-Glutamin, im Medium gegenüber bekannten Zellkulturmedien reduziert ist, in besonders bevorzugter Ausführungsform frei von Glutamin und/oder glutaminhaltigen Ersatzstoffen ist, und

b) die Konzentration von Pyruvat im Medium gegenüber bekannten Zellkulturmedien erhöht ist, wobei in einer bevorzugten Ausführungsform die Endkonzentration von Pyruvat und/oder pyruvathaltigen Stoffen im Zellkulturmedium insbesondere von 2,5 bis 100 mmol/l, bevorzugt von 5 bis 60 mmol/l beträgt und besonders bevorzugt von 5 mol/l bis 20 mol/l beträgt, insbesondere bevorzugt zwischen 5 mmol/l und 10 mmol/l liegt.

Die Erfinder fanden überraschend, dass durch die Kultivierung in dem Zellkulturmedium die Akkumulation von schädlichen Ammoniumionen im Zellkulturmedium reduziert, insbesondere beseitigt wird. Dabei fanden die Erfinder, dass besonders überraschend die zu erwartenden Mangelerscheinungen, welche erwartungsgemäß durch fehlendes Glutamin und/oder fehlende glutaminhaltige Stoffe in der Zellkultur ausgelöst werden, wie vermindertes Zellwachstum und/oder verringerte Produktbildung, durch eine gleichzeitig erhöhte Menge an Pyruvat und/oder pyruvathaltigen Stoffen im Zellkulturmedium verhindert wird. Besonders überraschend ist dabei, dass Pyruvat und/oder pyruvathaltige Stoffe Glutamin und/oder glutaminhaltige Stoffe in ihrer Wirkung im Zellkulturmedium ersetzen können. Durch den Einsatz von Pyruvat wird besonders vorteilhaft die Menge an von der Zellkultur freigesetztem Lactat reduziert. Der erfindungsgemäße Zusatz von Pyruvat und/oder pyruvathaltigen Stoffen dient damit auch, besonders vorteilhaft, dem teilweisen oder vollständigen Ersatz anderer Kohlenstoff- beziehungsweise Energiequellen wie Glucose und/oder anderer Zucker. Es zeigt sich also, dass Pyruvat eine gute Kohlenstoff- und Energiequelle für die tierische oder menschliche Zelle darstellt.

Durch die erfindungsgemäße Kombination eines im Wesentlichen glutaminfreien Mediums mit der Gegenwart von Pyruvat im Medium wird, besonders vorteilhaft, die Verminderung beziehungsweise die Beseitigung der beiden Hemmstoffe Ammonium und Lactat im Zellkulturmedium erreicht, was zu positiven Auswirkungen auf die Zellkulturen, insbesondere das Zellwachstum und die Produktbildung führt. Die bisher zum Zwecke der Beseitigung von Ammonium und/oder Lactat aus Zellkulturmedien eingesetzten technisch aufwendigen beziehungsweise Ressourcen-verbrauchenden und daher kostspieligen Verfahren werden im Zusammenhang mit dem vorgenannten Zellkulturmedium überflüssig.

Bei der Kultivierung von MDCK-Zellen oder CHO-Zellen konnte ein stabiles Wachstum über mehr als 5 Passagen sowie eine erfolgreiche Produktbildung nachgewiesen werden. Zelllinien von CHO-, BHK- und HeLa-Zellen wuchsen stabil über mehrere Passagen in statischen Zellkulturflaschen (zum Beispiel T75), wobei diese mehr als fünfmal mit einem Splittingverhältnis von cirka 1:3 bis 1:6 passagiert wurden. Allgemein wird ein Medium, das mehr als 5 Passagen ermöglicht, als dauhaft geeignet und wachstumsfördernd für Zellkulturen erachtet. Vor jeder Passage konnte dabei am Mikroskop beobachtet werden, dass die Zellen konfluent gewachsen waren. Demgemäß ist der positive Effekt, der in den nachfolgenden Beispielen, beispielhaft für MDCK-Zelllinien gezeigt wird, auch auf eine Vielzahl anderer Zellen übertragbar.

Das Fehlen von Glutamin führt, ohne auf die Theorie beschränkt zu sein, auch zu einer Anpassung des Stoffwechsels der kultivierten Zellen, insbesondere des Aminosäuremetabolismus. In entsprechend ausgestatteten reichhaltigen Medien wird der Stickstoffkreislauf auch über sehr lange Zeit aufrechterhalten.

Erfindungsgemäß bevorzugt wird das erfindungsgemäße Verfahren in Vorrichtungen durchgeführt, die geeignet sind, ideale Bedingungen für das Zellwachstum zu schaffen. Von besonderer Bedeutung sind dabei auch die Sterilität der Zellkultur sowie die Einstellung bestimmter Parameter wie beispielsweise Temperatur, pH-Wert, Osmolarität, Sauerstoffpartialdruck und Nährstoffangebot, sowie das Angebot an wachstumsstimulierenden Substanzen und Vitaminen. Die Sterilität wird in an sich bekannter Weise durch geeignete Steriltechniken, wie den Einsatz von Desinfektionsmitteln, die Verwendung steriler Arbeitsbänke und Arbeitsgeräte sowie das sterile Filtrieren und/oder Autoklavieren von Lösungen sichergestellt.

Im Folgenden werden vorteilhafte Kultivierungsbedingungen für die Kultivierung von Säugetierzellen mit dem erfindungsgemäßen Verfahren dargestellt; selbstverständlich sieht das erfindungsgemäße Verfahren bei der Kultivierung anderer tierischer Zellen, wie nicht-Säugetierzellen, und/oder modifizierter tierischer Zellen, auch andere von den unten genannten Parametern abweichende Kultivierungsbedingungen vor, soweit sie für die Kultivierung dieser Zellen nützlich sind: Die Temperatur von üblicherweise cirka 37°C für Säugetierzellen wird insbesondere in Brutschränken, Bruträumen und/oder im Bioreaktor durch Heizung beziehungsweise Kühlung bereitgestellt. Der pH-Wert des erfindungsgemäßen Zellkulturmediums und eventuell zusätzlicher für die Herstellung der Zellkultur verwendeter Medien beträgt üblicherweise pH 7,2 bis pH 7,3. In der erfindungsgemäßen Zellkultur werden bevorzugt vorwiegend Bicarbonatpuffer (Carbonat/Hydrogencarbonat) verwendet, welche auf $CO_2$-Begasung beziehungsweise $CO_2$-haltige Begasung beispielsweise mit Carbogen-Gas (95% $O_2$, 5% $CO_2$) ausgerichtet sind. Die $CO_2$-haltige Begasung findet bevorzugt in Brutschränken, Bruträumen und/oder im Bioreaktor statt. Neben der bevorzugten Einbringung von Kohlendioxid ist insbesondere auch die Einbringung von Sauerstoff in die Kultur vorgesehen, um einen günstigen Sauerstoffpartialdruck zu erhalten. Dies geschieht im einfachsten Fall durch Begasung des Gasraums oberhalb des Zellkulturmediums, insbesondere sind jedoch begaste Bioreaktoren zur Kultivierung vorgesehen, worin Sauerstoff und/oder Sauerstoff-haltiges Gas, wie Carbogen-Gas, direkt ins Medium eingebracht wird.

Erfindungsgemäß bevorzugt ist weiter vorgesehen, die zu kultivierenden Zellen, insbesondere Säugetierzellen, entweder adhärent an Gefäßoberflächen als statische Kulturen zum Beispiel in Kulturschalen oder in Suspension wachsen zu lassen. Für adhärente Zellen in Suspension werden zum Wachstum geeignete Oberflächen, beispielsweise Microcarrier wie Cytodex 1® der Firma Pharmacia eingesetzt. Insbesondere werden an solche Trägerpartikel adhärierte Zellen in gerührten Bioreaktoren oder Spinnerflaschen kultiviert. Bioreaktoren stellen dabei die bevorzugte Form für die industrielle Produktion dar.

Die Figuren zeigen:

Figur 1 Wachstumsversuche in verschiedenen Medien in 12-Well-Zellkulturplatten (Bebrütung: 7 Tage bei 37°C, 5 % $CO_2$), Aufnahmen mit cirka 160-facher Vergrößerung im Phasenkontrast,

Figur 2 Verlauf der Zellzahl bei der Kultivierung in Spinnerflaschen im erfindungsgemäß verwendeten Medium I (Versuchsgruppe A) und in Standard-GMEM (Kontrollguppe B),

Figur 3 Verlauf der Zellzahl in logarithmischer Darstellung bei der Kultivierung in Spinnerflaschen im erfindungsgemäß verwendeten Medium I (Versuchsgruppe A) und in Standard-GMEM (Kontrollguppe B),

Figur 4 Verlauf der Metabolite Glucose, Lactat, Glutamin und Ammonium über eine Kultivierungsdauer von 120 Stunden in Spinnerflaschen in Standard-GMEM,

Figur 5 Verlauf der Metabolite Glucose, Lactat und Pyruvat über eine Kultivierungsdauer von 120 Stunden in Spinnerflaschen im erfindungsgemäß verwendeten glutaminfreien Medium I,

Figur 6 Verlauf der Metabolite Glucose, Lactat, Glutamin und Ammonium über eine Kultivierungsdauer von über 168 Stunden im Bioreaktor in Standard-GMEM,

Figur 7 Verlauf der Metabolite Glucose, Lactat und Pyruvat über eine Kultivierungsdauer von über 168 Stunden im Bioreaktor im erfindungsgemäß verwendeten glutaminfreien Medium I,

Figur 8 Verlauf des Sauerstoffverbrauchs über eine Kultivierungsdauer von über 168 Stunden im Bioreaktor in Standard-GMEM,

Figur 9 Verlauf des Sauerstoffverbrauchs über eine Kultivierungsdauer von über 168 Stunden im Bioreaktor im erfindungsgemäß verwendeten glutaminfreien Medium I,

Figur 10 Fluoreszenzmikroskopische Aufnahmen von MDCK auf 3,3 g/l Cydodex 1 im erfindungsgemäß verwendeten Medium I (Versuchsgruppe A (VG A)) bei einer Kultivierungsdauer von t = 2 h, 17 h, 27 h, 41 h, 66 h und 88 h, Bildgröße: 651 x 651 $\mu$m,

Figur 11 Fluoreszenzmikroskopische Aufnahmen von MDCK auf 3,3 g/l Cydodex 1 in Standard-GMEM (Kontrollgruppe B (KG B)) bei einer Kultivierungsdauer von t = 17 h, 27 h, 41 h, 66 h, 76 h und 88 h, Bildgröße: 651 x 651 $\mu$m.

Beispiel 1: **Glutaminfreies Medium**

Ammonium hat vielfältige negative Einflüsse auf Zellen wie Zellwachstum und Produktbildung. Mit dem Ziel, die negativen Einflüsse des während der Kultivierung gebildeten Ammoniums zu beseitigen, wurde ein neu entwickeltes glutaminfreies Medium eingesetzt. Dieses Medium dient insbesondere der Kultivierung von MDCK-Zellen und basiert auf einem Glasgow-Minimal-Essential-Medium (GMEM) der Firma Gibco (Invitrogen) oder der Firma Biochrom. Dabei werden Pulvermedien oder Flüssigmedien verwendet, worin gegenüber dem eigentlichen GMEM die Komponente L-Glutamin fehlt und zusätzlich Pyruvat in Form von Natriumpyruvat in einer Endkonzentration von 10 mmol/l (=1,1 g/l) (Medium I). Zu dem Medium wird zusätzlich 10 % fötales Kälberserum (FCS) und 0,2 % Pepton zugefügt. Im Einzelnen weist ein erfindungsgemäß verwendetes Medium folgende Zusammensetzung auf (Konzentrationen in mmol/l):

Anorganische Salze:

| | |
|---|---|
| Calciumchlorid ($CaCl_2$) | 1,80 |
| Eisennitrat ($Fe(NO_3)_3\ 9H_2O$) | 0,0025 |
| Kaliumchlorid (KCl) | 5,33 |
| Magnesiumsulfat ($MgSO_4\ 7H_2O$) | 0,814 |
| Natriumchlorid (NaCl) | 110,34 |
| Natriumhydrogencarbonat ($NaHCO_3$) | 32,74 |
| Natriumphosphat ($NaH_2PO_4\ H_2O$) | 0,899 |

Aminosäuren:

| | |
|---|---|
| L-Arginin-HCl | 0,200 |
| L-Cystin-2HCl | 0,100 |
| L-Histidin-HCl-$H_2O$ | 0,100 |
| L-Isoleucin | 0,400 |
| L-Lysin-HCl | 0,400 |
| L-Methionin | 0,101 |
| L-Phenylalanin | 0,200 |
| L-Threonin | 0,400 |
| L-Tryptophan | 0,0392 |
| L-Tyrosin-2Na-2$H_2O$ | 0,200 |
| L-Valin | 0,400 |

Vitamine:

| | |
|---|---|
| D-Calciumpantothenat | 0,0042 |
| Cholinchlorid | 0,0143 |
| Folsäure | 0,0045 |
| i-Inositol | 0,0200 |
| Niacinamid | 0,0164 |
| Pyridoxal-HCl | 0,0098 |
| Riboflavin | 0,0005 |
| Thiamin-HCl | 0,0059 |

Andere Komponenten:

| | |
|---|---|
| D-Glucose | 25,00 |

(fortgesetzt)

| Andere Komponenten: | |
| --- | --- |
| Phenolrot | 0,0377 |
| **Natriumpyruvat** | **10,00 (= 1100 mg/l)** |
| Pepton, 20 %ig: | 1 Vol.-% |
| FCS: | 10 Vol.-% |

Beispiel 2: **Kultivierung von MDCK-Zellen in Glutamin-defizienten Medien**

a) Verwendete Medien

Für Wachstumsversuche in Zellkulturplatten wurde ein Glutamindefizientes GMEM eingesetzt, worin an Stelle von Glutamin Pyruvat, Glycerin und/oder Citrat eingesetzt wurde oder aber zusätzlich zu Glutamin Pyruvat, Glutamin und/oder Citrat eingesetzt wurde. Die folgende Tabelle zeigt die Zusammensetzungen der verwendeten Medien bezüglich ihrem Gehalt an Glutamin, Pyruvat, Glycerin und/oder Citrat in mmol/l:

| Medium | Glutamin | Pyruvat | Glycerin | Citrat | Wachstum |
| --- | --- | --- | --- | --- | --- |
| Medium a | - | - | - | - | - |
| Medium b | - | - | 9,8 | | - |
| Medium c | - | - | - | 1,24 | - |
| Medium d | - | 2,5 | - | - | + |
| Medium e | - | 5,0 | - | - | + |
| Medium f | - | 9,8. | - | - | + |
| Medium g | - | 19,2 | - | - | + |
| Medium h | - | 5,0 | 5,0 | - | + |
| Medium i | - | - | 5,0 | 2,5 | - |
| Medium k | - | 5,0 | - | 2,5 | + |
| Medium l | - | 5,0 | 5,0 | 2,5 | + |
| Medium m | 4,0 | 5,0 | 5,0 | - | + |
| Medium n | 4,0 | - | 5,0 | 5,0 | - |
| Medium o | 4,0 | 5,0 | - | 2,5 | + |
| Medium p | 4,0 | 5,0 | 5,0 | 2,5 | + |
| + = Wachstum; - = kein Wachstum | | | | | |

b) Die Wachstumsversuche von MDCK-Zelllinien

In den entsprechenden Medien wurden MDCK-Zellen (ATCC Nr. 84121903) für 7 Tage in 12-Well-Zellkulturplatten mit einem Volumen von jeweils 2 ml bei 37°C und 5 % $CO_2$-Sättigung bebrütet. Dabei wurden zu Versuchsbeginn cirka $1 \times 10^4$ Zellen pro ml eingesetzt. Anschließend wurde die Zellvermehrung bei cirka 160-facher Vergrößerung unter dem Mikroskop im Phasenkontrast untersucht.

c) Ergebnisse

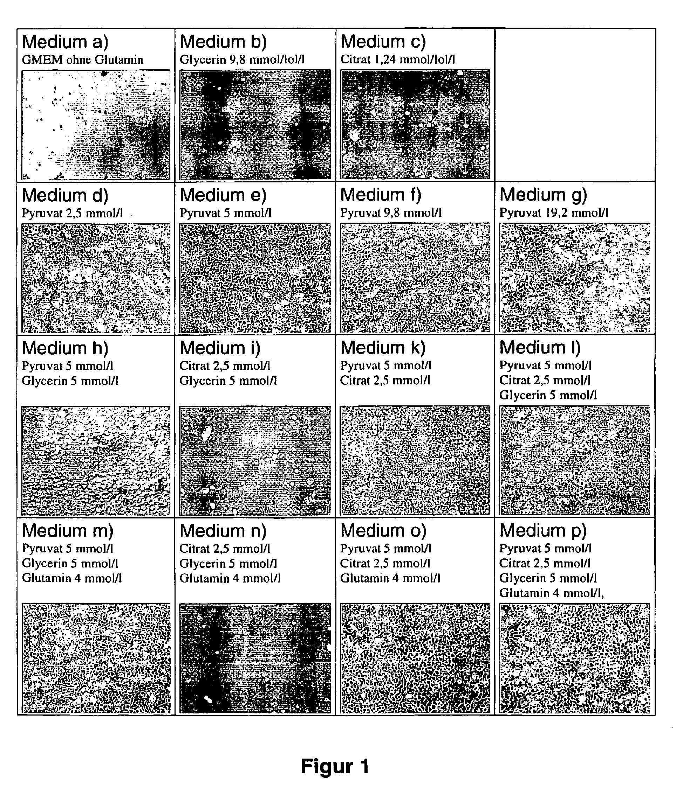

Figur 1 zeigt die Ergebnisse der Wachstumsversuche in den entsprechenden Medien a) bis p). Nur wenn Pyruvat im Medium vorhanden war, zeigte sich Zellwachstum. In den Kulturplatten wuchsen die Zellen in den Medien d, e, f, g, h, k, l, m, o und p bis zur Konfluenz. In allen Ansätzen ohne Pyruvat blieb erkennbares Wachstum aus.

Beispiel 3: **Kultivierung von MDCK-Zellen in glutaminfreiem, pyruvathaltigen Medium in gerührten Kultursystemen**

a) Kultivierung der Zellen auf Microcarrier

MDCK-Zellen (ATCC Nr. 84121903) wurden in gerührten Kultursystemen, in sogenannten Spinnerflaschen angezogen. Bei den verwendeten Spinnerflaschen handelt es sich um Glasflaschen mit einer Rührvorrichtung und mehreren separaten Zugängen. Spinnerflaschen der Firma Wheaton hatten zwei Zugänge, ein Arbeitsvolumen von jeweils 250 ml und einen schwebenden Rührer mit zwei zusätzlichen Rührblättern. Der Zugang war mit einem Membranfilterdeckel verschlossen, um den Gasaustausch mit der Umgebung zu ermöglichen. Der zweite Zugang diente zur Probenentnahme und hatte einen geschlossenen Deckel. Die Spinnerflaschen wurden in einem Brutschrank gerührt, welcher auf 37°C und 5 % $CO_2$-Sättigung eingestellt war.

Als Carrier, das heißt als Microcarrier, woran die kultivierten MDCK-Zellen adhärierten, wurde Cytodex 1® der Firma Pharmacia verwendet. Dieser weist eine Dichte in Saline von 1,03 g/ml auf. Folgende Größenverteilung war charakteristisch: Mittlere Größe: 180 $\mu$m, Größenverteilung (5 %- bis 95 %-Perzentil): 131 bis 220 $\mu$m, durchschnittliche Oberfläche in Saline: 4400 $cm^2$/g (Trockensubstanz), durchschnittliche Carriermenge/g (Trockensubstanz): 6,8 x $10^6$. Die Microcarrier wurden vor den Versuchen entsprechend der Herstellerangaben vorbereitet: Eine bekannte Menge von Cytodex 1® wurde in phosphatgepufferter Saline (PBS) zunächst mindestens drei Stunden quellen lassen, danach dreimal mit PBS gewaschen und anschließend autoklaviert. Danach wurde der PBS-Überstand gegen steriles Medium ausgetauscht.

Um die Anheftung der Carrier an die Glaswand der Spinnerflaschen zu verhindern, wurden die Spinnerflaschen vor dem ersten Gebrauch durch Einbrennen von Silikonöl bei cirka 100°C silikonisiert.

Die Spinnerflaschen und das Zubehör wie Filterdeckel und Rührer wurden vor dem Einsatz mit geeigneten Mittels sterilisiert, das heißt autoklaviert. Medien und Carrier wurden vor Versuchsstart eingefüllt und cirka 1 Stunde bei 37°C vorinkubiert. Während dieser Zeit wurden die cirka 4 bis 7 Tage alten MDCK-Zellen, welche zuvor beispielsweise in Rollerflaschen kultiviert wurden, geerntet und gezählt. Mit der Zugabe der Zellen in die Spinnerflaschen startete der Versuch. Für alle Versuche wurde eine gleiche Zellcharge (evtl. unterscheidliche Passagezahl) verwendet.

Die Flaschen wurden von Beginn an mit 30 Umdrehungen pro Minute gerührt, nach cirka 24 Stunden wurde die Drehzahl auf 50 Umdrehungen pro Minute erhöht; dies reichte aus, um die Carrier gleichmäßig zu suspendieren. Die Wachstumsversuche dauerten 5 bis 6 Tage (120 beziehungsweise 144 Stunden). Nach dem Versuchsstart wurde im Abstand von cirka 12 Stunden jeweils eine Probe aus der Carrierkultur genommen.

b) Zellzahlbestimmung

Die Proben für die Zellzahlbestimmung wurden aus einer homogenen Suspension von Carriern mit einer 5 ml-Pipette entnommen. Bei jeder Probenentnahme bestimmte man die Lebendzellzahl auf den Carriern. Zusätzlich wurde der Überstand auf tote Zellen untersucht. Die Zellzahl wurde dabei durch Auszählen der Zellen mit einem Hämocytometer oder einer Fuchs-Rosental-Zählkammer mit einem Mikroskop mit Phasenkontrastoptik und cirka 100-facher Endvergrößerung durchgeführt. Adhärent wachsende Zellen konnten nicht unmittelbar mit dem Hämocytometer gezählt werden; um freie Zellen zu erhalten, löste man die Zellen aus den Carrierkulturen beispielsweise mit 10 x Trypsinlösung (2,5 g/l porcines Trypsin, 1,0 g/l EDTA).

c) Bestimmung der Medienparameter und der extrazellulären Metaboliten

Aus den entnommenen Carrierkulturen wurden im Mediumüberstand pH-Wert, die Konzentration von Glucose, Lactat, Pyruvat, Glutamin und Ammonium bestimmt. Direkt nach der Probennahme wurden die Proben entweder sofort gemessen oder 3 min bei 80°C erhitzt und zur späteren Messung eingefroren. Die Konzentrationen von Glucose und Lactat wurden mit dem automatischen, biochemischen Analysator YSI 2700 Select® der Firma YSI Inc. durchgeführt. Das Messprinzip beruht auf enzymatischen Reaktionen, die jeweils spezifisch für Glucose beziehungsweise Lactat sind. Die Enzyme sind jeweils zwischen einer Polycarbonat- und einer Zelluloseacetatschicht immobilisiert. Bei der Reaktion wird Wasserstoffperoxid gebildet und an einer Platinelektrode oxidiert. Die an der Elektrode abgreifbare Spannung ist der Konzentration des Analyten proportional. Die Medienüberstände wurden dabei entsprechend der zu erwartenden Konzentration pur oder 1:2 mit Wasser verdünnt gemessen. Das Messvolumen betrug jeweils 10 $\mu$l.

Für die biosensorische Bestimmung von Glutamin im Medium wurde der automatische, biochemische Analysator YSI 7100 MBS von YSI Inc. eingesetzt. Dabei wird Glutamat und Glutamin in entsprechenden enzymatischen Reaktionen umgesetzt, die bei diesen Reaktionen entstehenden Nebenprodukte nachgewiesen und angezeigt (siehe oben).

Für die Bestimmung der Ammoniumionenkonzentration ($c(NH_4^+)$) in den Medienproben wurde das automatische chemische Analysegerät Vitros DT-II® von Ortho Clinical Diagnostics eingesetzt. Die Nachweisreaktion beruht auf einer Umsetzung von Ammoniak ($NH_3$) mit Bromphenolblau, wobei ein blauer Farbkomplex entsteht, der bei einer Wellenlänge von 605 nm detektiert werden kann. Das Probenvolumen betrug 10 μl. Jede Probe wurde vor der Messung 1:2 beziehungsweise 1:10 mit PBS verdünnt, um innerhalb des Messbereichs zu liegen und um störende Einflüsse des Mediums zu minimieren.

Zur Bestimmung der Pyruvatkonzentration im Medium wurde Pyruvat mit NADH + $H^+$ in einer spezifischen Reaktion von Lactatdehydrogenase (LDH) äquimolar zu Lactat und $NAD^+$ umgesetzt. Die Abnahme der NADH-Konzentration vom Startpunkt vor LDH-Zugabe bis zum Endpunkt der Reaktion, wenn Pyruvat vollständig umgesetzt war, wurde anhand der Extinktion bei 340 nm beobachtet und war ein direktes Maß für die Pyruvatkonzentration. Mediumsproben wurden vor der Messung entsprechend mit Wasser verdünnt (z.B. 1:25), um im Messbereich zu liegen (Methode modifiziert nach Bergmeyer, H.U: Methoden der enzymatischen Analyse, Verlag Chemie Weinheim/Bergstr. 1974, 1491-1496).

d) Ergebnisse

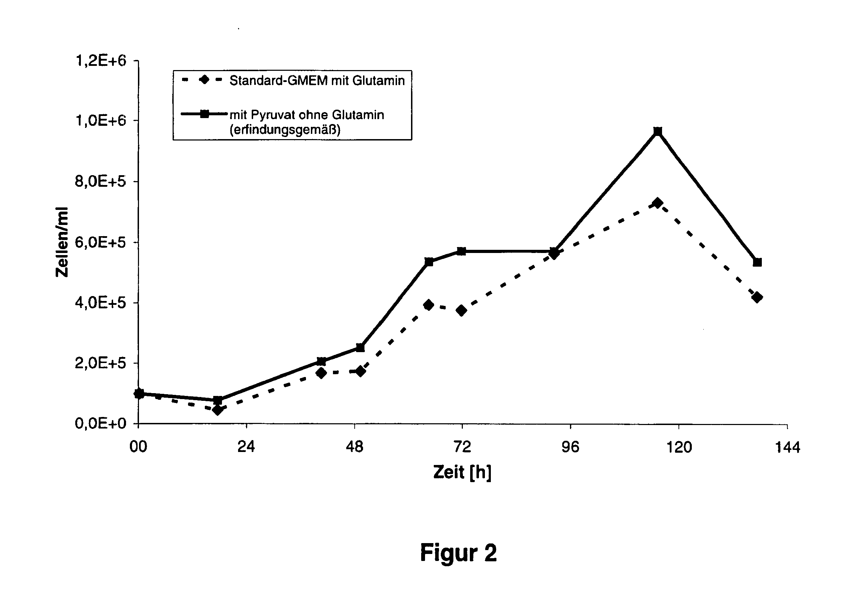

In den Figuren 2 bis 6 sind die Ergebnisse der Kultivierungsversuche in Spinnerflaschen dargestellt. In der Versuchsgruppe A (erfindungsgemäß) wurden die Zellen unter den vorgenannten Standardbedingungen in dem erfindungsgemäß verwendeten glutaminfreien Medium I (siehe Beispiel 1) kultiviert. In einer Kontrollgruppe B wurden die Zellen unter denselben Bedingungen in einem bekannten Standard GMEM, welches 2 mmol/l Glutamin enthielt, kultiviert.

Figur 2 zeigt das Ansteigen der Zellzahlen in den beiden Versuchsgruppen über die Zeit. Die Zellvermehrung ist bei der Versuchsgruppe A (erfindungsgemäß) gegenüber der Kontrollgruppe B nach einer Kultivierungszeit von cirka 5 Tagen (cirka 120 Stunden) deutlich erhöht. Die Produktion von $NH_4^+$ blieb im erfindungsgemäß verwendeten Medium fast vollständig aus. Nach cirka 120 Stunden Kultivierungsdauer lag der Gehalt an $NH_4^+$ im Kulturmedium der Versuchsgruppe A bei 0,23 mmol/l. Demgegenüber betrug die $NH_4^+$-Konzentration in der Kontrollgruppe B zum gleichen Zeitpunkt bereits 2,5 mmol/l.

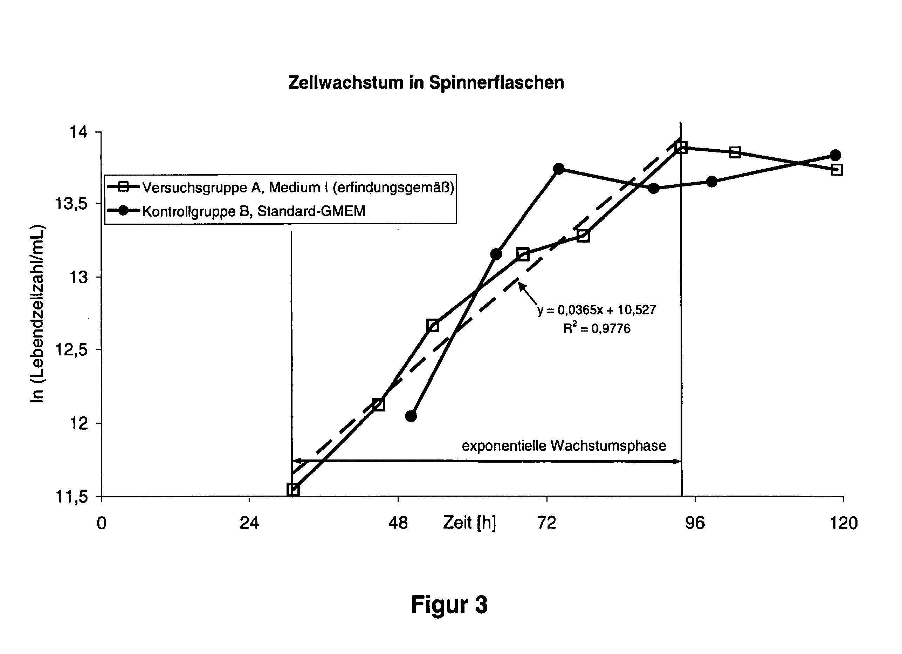

Figur 3 zeigt die Zellkonzentration in logarithmischer Darstellung im zeitlichen Verlauf von Experimenten in Spinnerflaschen, einmal nach Bedingungen von Versuchsgruppe A in Medium I (Beispiel 1), einmal nach Bedingungen von Kontrollgruppe B in Standard-GMEM. Die exponentielle Wachstumsphase und die Endzellkonzentration sind in beiden Gruppen sehr ähnlich und führen jeweils zu einer Zelldichte von ca. $1 \times 10^6$ Zellen/ml nach ca. 4 Tagen. Für den Graphen der Versuchsgruppe A ist eine lineare Regressionsgerade der Form:

$$\ln (\text{Zelldichte [Zellen/ml]}) = \mu\, t + C$$

angepasst. Es ergibt sich eine Wachstumsrate $\mu$ = 0,0365 h$^{-1}$, entsprechend einer Verdopplungszeit $t_D$ = ln2 / $\mu$ = 19 Stunden. Versuchsgruppe A zeigt damit eindeutig schnelles Zellwachstum ohne Einbußen gegenüber konventionelle Zellkulturmedien.

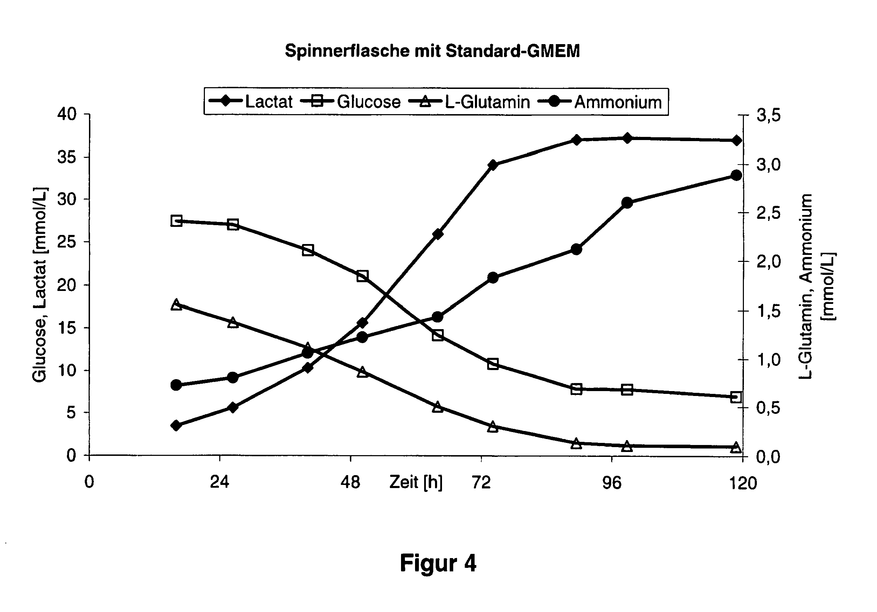

Figur 4 zeigt den zeitlichen Verlauf von Glucose, Lactat, Glutamin und Ammonium in Kontrollgruppe B. Es werden 20,5 mmol/l Glucose verbraucht und 37 mmol/l Lactat akkumuliert. Glutamin wird im Kulturverlauf vollständig verbraucht. Es werden 2,9 mmol/l Ammonium ins Medium abgegeben.

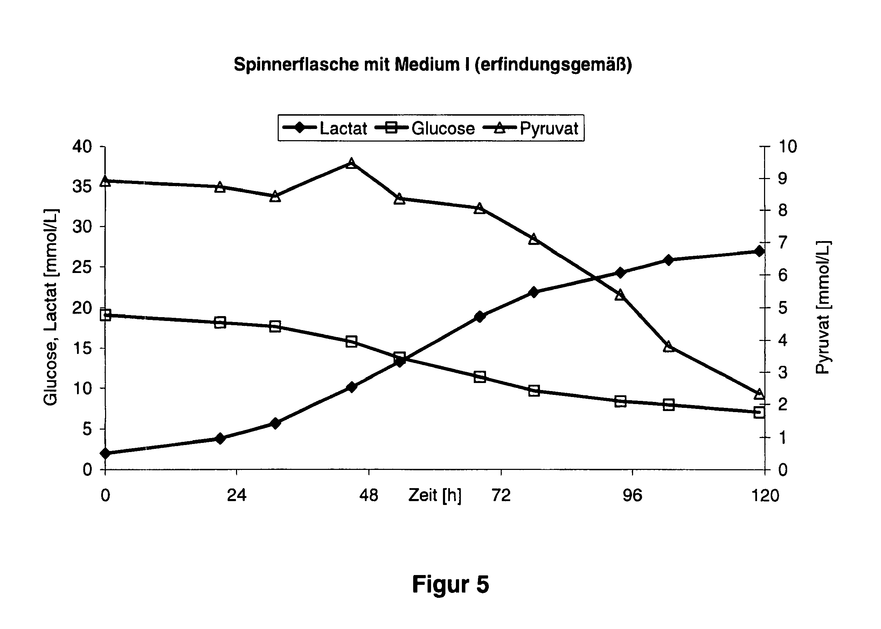

Figur 5 zeigt Glucose, Lactat und Pyruvat der Versuchsgruppe A (erfindungsgemäß). Es werden nur 15 mmol/l Glucose verbraucht und die maximale Lactatkonzentration von 27 mmol/l und bleibt mit 10 mmol/l unter der maximalen Lactatkonzentration der Kontrollgruppe B. Der Pyruvatverbrauch von 6,7 mmol/l zeigt, dass Pyruvat eine hervorragende Kohlenstoff- und Energiequelle für die Zellen ist. Bezogen auf die Kohlenstoffmenge werden 20 mmol/l Kohlenstoff aus dem C-3-Körper Pyruvat verwertet. Demgegenüber stehen ca. 10 mmol/l Kohlenstoff aus dem C-5-Körper Glutamin bei 2 mmol/l Anfangskonzentration nach Rezept. Die Ergebnisse legen nahe, dass Pyruvat, besser als Glutamin, zumindest einen Teil der Glucose im Medium ersetzen und deren Rolle als Energie- und Kohlenstoffquelle übernehmen kann. Der Gesamtverbrauch an Glucose ist im erfindungsgemäß verwendeten Medium I geringer.

Der Glucoseumsatz bzw. -durchsatz durch die Glycolyse zu Lactat ist in Säugerzellen abhängig von der Glucosekonzentration im Medium, bei hohen Glucosekonzentrationen hoch, bei niedrigen Glucosekonzentrationen niedrig, (Glacken et al., 1986, Biotechnol. Bioeng. 32, 1015-1028). Gemäß den gefundenen Ergebnissen kann die Anfangskonzentration von Glucose in Medien folglich gesenkt werden, um eine noch geringere Lactatproduktion zu erhalten.

Beispiel 4: **Virusproduktion in Glutamin-defizienten Medien**

a) Kultivierung in Zellkulturflaschen und Infektion

Um den Effekt der Kultivierung in glutaminfreien Medien auf die Virusproduktion infizierter Zellkulturen zu untersuchen, wurden MDCK-Zellen in Zellkulturflaschen kultiviert und mit Influenzavirus infiziert.

MDCK-Zellen (ATCC Nr. 84121903) wurden in statischen Kulturen in Zellkulturflaschen (z.B. T 75) bei 37°C und 5 % $CO_2$-Sättigung kultiviert. Im Abstand von 4 bis 8 Tagen wurden die Zellen passagiert. Dabei löste man die Zellen mit 1 x Trypsinlösung von den Kulturgefäßen ab und brachte sie 1:4 bis 1:10 verdünnt in eine neue Kulturflasche mit frischem Medium. Die Passagierung wurde nach folgendem Schema durchgeführt:

- Medium aus den Kulturgefäßen in steriles Abfallgefäß abgießen

- Flasche zweimal mit sterilem PBS bei Raumtemperatur waschen

- je nach Größe der Kulturflasche 1 x Trypsin/EDTA-Lösung (0,25 g/l porcines Trypsin, 0,1 g/l EDTA) zugeben (ca. 1 ml für 25 $cm^2$-Flaschen)

- 20 Minuten bei 37°C inkubieren, dabei die Zellen zwischendurch und besonders am Ende von der Gefäßwand abklopfen

- FCS zugeben, um Trypsinwirkung zu hemmen

- 1/4 bis 1/10 der Zellsuspension in eine neue Flasche mit frischem Medium überführen und die Flasche in den Brutschrank stellen.

Alle MDCK-Zellen wurden für mehrere Passagen (mehr als 3) im erfindungsgemäß verwendeten Medium I (Beispiel 1) kultiviert. Anschließend wurden zwei Versuchsgruppen gebildet. Versuchsgruppe A wurde in Medium I kultiviert, Kontrollgruppe B in Standard-GMEM mit 2 mmol/I Glutamin. Nach einer 4-tägigen Inkubation waren beide Kulturen in statischen T 75 Zellkulturflaschen (Kulturvolumen ca. 50 ml) konfluent angewachsen.

b) Infektion

Die Zellen wurden danach weitergezüchtet und dabei mit dem Virus "Equine Influenza" Stamm A/Equi2 Nr. H3N8, Newmarket 1/93 (NIBSC, Potters Bar, London) infiziert. Das Medium wurde dazu jeweils abgegossen und die Zellen wurden dreimal mit PBS (Raumtemperatur) gewaschen, um alle Serumbestandteile bestmöglich zu entfernen. Danach wurde Versuchsgruppe A mit 50 ml Medium 1 allerdings ohne FCS, aber mit 0,2% Pepton, 10 $\mu$g/ml Trypsin und 0,1 Vol-% eines influenzahaltigen Kulturüberstandes infiziert. Kontrollgruppe B wurde mit 50 ml Standard-GMEM allerdings ohne FCS, aber mit 0,2% Pepton, 10 $\mu$g/ml Trypsin und 0,1 Vol-% eines influenzahaltigen Kulturüberstandes ebenfalls infiziert.

Die Kulturen wurden weiter bei 37°C, 5% $CO_2$ inkubiert. Am Tag 1, Tag 2 und Tag 4 nach der Infektion wurden Proben des Medienüberstandes genommen und bei -70°C eingefroren.

Von allen sechs Proben wurde nach Versuchsende ein Hämagglutinationstest durchgeführt.

c) Hämagglutinationstest

Der Hämagglutinationstest (HA-Test) ist eine schnelle und einfache Methode, um Influenzaviren in einer Zellkultur zu quantifizieren. Der Test nutzt aus, dass Influenzaviren durch die Bindung an die Oberflächen roter Blutzellen diese agglutinieren können. Beim HA-Test wurden rote Hühnerblutzellen mit verschiedenen Virusverdünnungen titriert. Von jeder Probe wurde eine Verdünnungsreihe mit 12 Verdünnungsstufen im PBS hergestellt. Im ersten Well wurde die Lösung pur vorgelegt (Verdünnungsfaktor 1), im zweiten war die Verdünnung 50 % (Verdünnungsfaktor 2), im dritten 25 % (Verdünnungsfaktor 4) usw. bis Verdünnungsfaktor 2048. Wenn genügend Virus in der Testlösung ist, agglutinieren die Zellen. Bei unterschwelligen Mengen an Virus findet keine Agglutination statt. Der HA-Test wird in 96-Well-Mikrotiterplatten mit rundem Boden durchgeführt. Die nicht-agglutinierten Zellen sinken auf den Boden der Platte und bilden einen roten Erythrozytenknopf, während agglutinierte Zellen eine rötlich-trübe Lösung im Well bilden. Zu jedem Well der Verdünnungsreihe mit jeweils 200 $\mu$l in PBS verdünnter Virusprobe wurden jeweils 100 $\mu$l einer Erythrozytengebrauchslösung mit cirka $10^7$ Erythrozyten pro ml gegeben und für 60 bis 90 Minuten bei Raumtemperatur inkubiert bis Erythrozytenknöpfe in den Wells deutlich zu sehen waren.

Die Erythrozytengebrauchslösung wurde wie folgt hergestellt:

-   In Falkonröhrchen wurden 5 ml kalte Alsevers-Lösung (Firma Gibco) mit jeweils 5 ml frischem Hühnerblut gemischt und auf Eis gelagert.

-   Zum Waschen der Zellen werden 1 ml der Blutsuspension in 50 ml-Falkonröhrchen mit jeweils 20 ml PBS gemischt und 5 Minuten bei 500 g zentrifugiert (schwache Bremse) und der Überstand vorsichtig dekantiert; das Waschen wurde einmal wiederholt.

-   Die Erythrozytenlösung wird auf ein Volumen von 40 ml aufgefüllt, die Erythrozytenkonzentration durch Auszählen bestimmt und die Gebrauchslösung auf eine Zahl von cirka $10^7$ Erythrozyten pro ml eingestellt.

-   Die Erythrozytengebrauchslösung wurde bei 4°C gelagert und maximal bis 14 Tage nach Herstellung verwendet.

Bei jedem Test wurde als interner Standard eine Viruslösung mit genau bekanntem HA-Wert mitgemessen. Die prozentuale Abweichung des HA-Wertes des Standards gegenüber dem bekannten HA-Wert des Standards wurde in alle übrigen Messwerte der gleichen Serie als Korrektur eingerechnet.
Die Endpunkttitration ergab den HA-Wert, das heißt das Well vor dem ersten Erythrocytenknopf enthielt per Definition die Virusmenge 1 HA-Unit. Der Verdünnungsfaktor des entsprechenden Wells gab die HA-Units pro 100 $\mu$l der Ausgangslösung an. Dieser Wert wird entweder direkt als HA-Units oder in Form des dekadischer Logarithmus der HA-Units angegeben.

d) Ergebnisse

Vor dem Infektionsversuch konnten MDCK-Zellen über mehr als drei Passagen in Medium I (erfindungsgemäß) kultiviert werden, ohne dass Veränderungen an den Wachstumseigenschaften festgestellt wurden. Eine Adaption der Zellen, die zuvor in Standard-GMEM kultiviert worden waren an das Medium I war nicht erforderlich.
Die Zellen konnten nach den Passagen in Medium I sowohl in Medium I wie auch in Standard-GMEM ohne weitere Adaption erfolgreich kultiviert und mit Influenzavirus infiziert werden.

Tabelle 1:

| [$\log_{10}$ (HA-Units)] | Tag 1 | Tag 2 | Tag 4 |
|---|---|---|---|
| Versuchsgruppe A: HA mit Medium I (erfindungsgemäß) | 176 | 1,93 | 2,25 |
| Kontrollgruppe B: HA mit Standard-GMEM | 1,76 | 1,93 | 1,93 |

Tabelle 1 zeigt die gemessenen HA-Werte bei Infektionsversuchen in T75-Flaschen (75 cm$^2$, 50 ml) bei Versuchsgruppe A und Kontrollgruppe B als dekadischen Logarithmus. Nach einer zweitägigen Infektionsphase wurde in beiden Versuchsgruppen gleichermaßen hohe Titer von Influenzavirus produziert. Bei der Versuchsgruppe A (erfindungsgemäß) hielt die Virusproduktionsphase länger an und es wurde bis zum Tag 4 nach Infektion deutlich mehr, nämlich doppelt so viel, Influenzavirus produziert wie in Kontrollgruppe B, wo sich der Virustiter von Tag 2 zu Tag 4 nicht mehr veränderte.

Beispiel 5: **Kultivierung von MDCK-Zellen in Glutamindefizientem Medium im Bioreaktor**

a) Kultivierung und Infektion

Es wurde ein Bioreaktor des Typs Biostat C® der Firma B. Braun Biotech eingesetzt. Der Bioreaktor hatte ein Arbeitsvolumen von 5 l und wurde von zwei Marine-Typ Rührern gerührt. Die Kultivierungen wurden dabei über das Prozessleitsystem PC S7 (Siemens) gesteuert. Der Reaktor war jeweils mit einer Zugabevorrichtung und einer Probenentnahme ausgestattet, welche jeweils getrennt und während des Kultivierungsbetriebs sterilisiert werden konnte. Alle im Zusammenhang mit dem Bioreaktor verwendeten Gefäße und Geräte, welche mit der Zellkultur in Berührung kamen, wurden vor und während den Experimenten stets sterilisiert.
Der Standardkultivierungsprozess bestand in einer Kultivierung einer 6 bis 7 Tage alten Vorkultur, welche in Rollerflaschen mit Standard GMEM-Pulvermedium kultiviert wurde. Der Kulturmaßstab lag bei cirka 4,8 l. Vom Beginn an wurde mit 50 Umdrehungen pro Minute gerührt. Temperatur und pH-Wert wurden über den gesamten Kulturverlauf konstant bei 37°C beziehungsweise zwischen pH 7,1 und pH 7,3 gehalten. Der Sauerstoffpartialdruck wurde durch Zuleiten von reinem Sauerstoff über 40 %, nach Möglichkeit zwischen 60 und 40 % gehalten. Die Carrierkonzentration betrug 3,3 g/l. Zur Kultivierung im Bioreaktor wurden in einem Versuchsansatz A (erfindungsgemäß) MDCK-Zellen in dem erfindungsgemäß verwendeten glutaminfreien Medium I kultiviert. Eine Adaptation der Zellen von GMEM der Vorkultur auf Medium I war nicht erforderlich.

In einem Kontrollansatz B wurde die gleiche Zelllinie aus derselben Vorkultur am gleichen Tag unter den gleichen Bedingungen in einem Standard-GMEM kultiviert. Nach vier Kulturtagen wurde das Medium abgelassen und die Carrier wurden 5 mal mit je ca. 3 l PBS gewaschen. Nach dem Waschen wurde frisches Infektionsmedium enthaltend 10 ml Influenzavirus (siehe Beispiel 4b)) (filtrierter Überstand aus einer vorher infizierten Kultur) und 10 $\mu$g/ml (Endkonzentration) procines Trypsin zugegeben. Am dritten Infektionstag, nachdem im Bioreaktor kein Sauerstoff mehr verbraucht wurde, beendete man den Versuch durch Inaktivieren des Virus. Der Reaktor wurde dazu mit HCl angesäuert (pH ca. 3) und eine Stunde auf 90 °C erhitzt. Die Kulturbrühe wurde danach verworfen.

Die Bestimmung der Medienparameter und der extrazellulären Metaboliten erfolgte gemäß Beispiel 3 c).

Zur Kontrolle der konfluenten Überwachung der Microcarrier mit den kultivierten Zellen wurden die Zellkerne der auf den Microcarriern fixierten Zellen mit dem fluoreszierenden Kernfarbstoff Sytox®green gefärbt. Die Aufnahmen wurden an einem konfokalen Laser Scanning Mikroscop Axiovert 100M (Zeiss) mit der Software LMS 5 (Zeiss) angefertigt. Die Bilder sind übereinander projizierte Schnitte der Z-Ebene. In der Projektion wird der gesamte Carrier erfasst. Die Aufnahmen geben Auskunft über den Grad der konfluenten Bewachsung der Carrier.

b) Ergebnisse

*Medienparameter und extrazelluläre Metaboliten*

Die Bestimmung der Medienparameter und der extrazellulären Metaboliten erfolgte gemäß Beispiel 3 c).

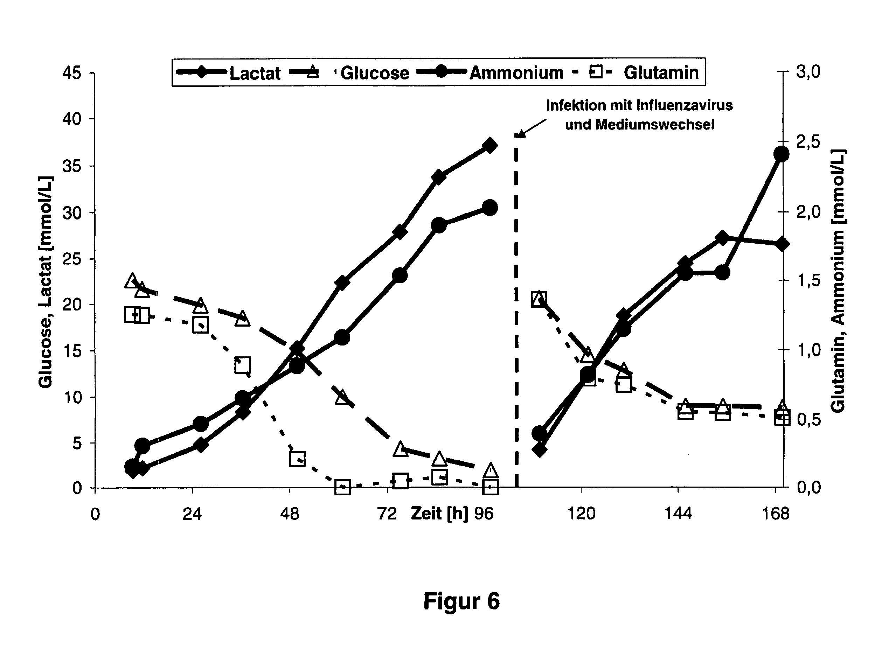

Figur 6 zeigt den Verlauf der Metabolite Glucose, Lactat, Glutamin und Ammonium im Kontrollansatz B über eine Kultivierungsdauer von 7 Tagen (168 Stunden). Nach einer Kultivierungsdauer von 4 Tagen wurden bei einem anfänglichen Glucosegehalt von 19,4 mmol/l cirka 35,6 mmol/l Lactat gebildet. Dies entspricht einer Lactatausbeute von 89 % Lactat pro Glucose (C-Molbasis). Am Ende der Zellanzucht war die Glucose fast vollständig verbraucht. 1,25 mmol/l Glutamin waren in dieser Kultur bereits nach etwa 2,5 Tagen verbraucht. Die Ammoniumkurve zeigt, dass etwa 1,7 mmol/l Ammonium gebildet wurden. Die Konzentration an Ammonium stieg auch nach dem Verbrauch des Glutamins weiter an. Die Stickstoffbilanz erlaubt, dass pro Molekül Glutamin zwei Moleküle Ammonium entstehen. Neben Glutamin waren aber auch eventuell weitere Aminosäuren an der Ammoniumproduktion beteiligt. Zum Zeitpunkt der Infektion mit Influenzaviren nach etwa 4 Tagen (104 Stunden) lag die Konzentration von Ammonium im Medium bei 2 mmol/l. Nach der Infektion wurden hohe Umsatzraten der Metabolite beobachtet. Der Umsatz der Metabolite sank, bis die Zellen nach zwei Infektionstagen keine Stoffwechselaktivität mehr zeigten.Der Ammoniumgehalt stieg noch weiter an als alle anderen Metabolite bereits unverändert blieben.

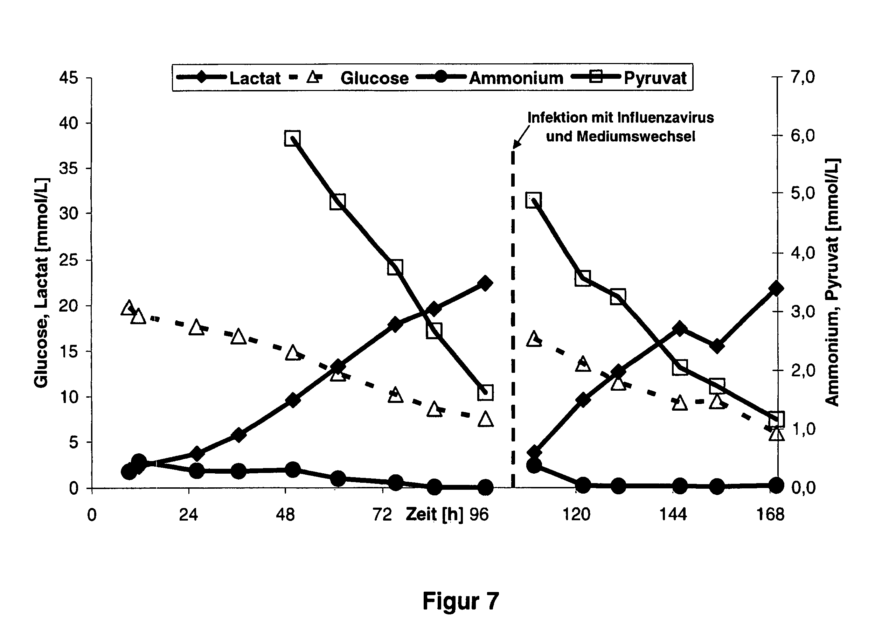

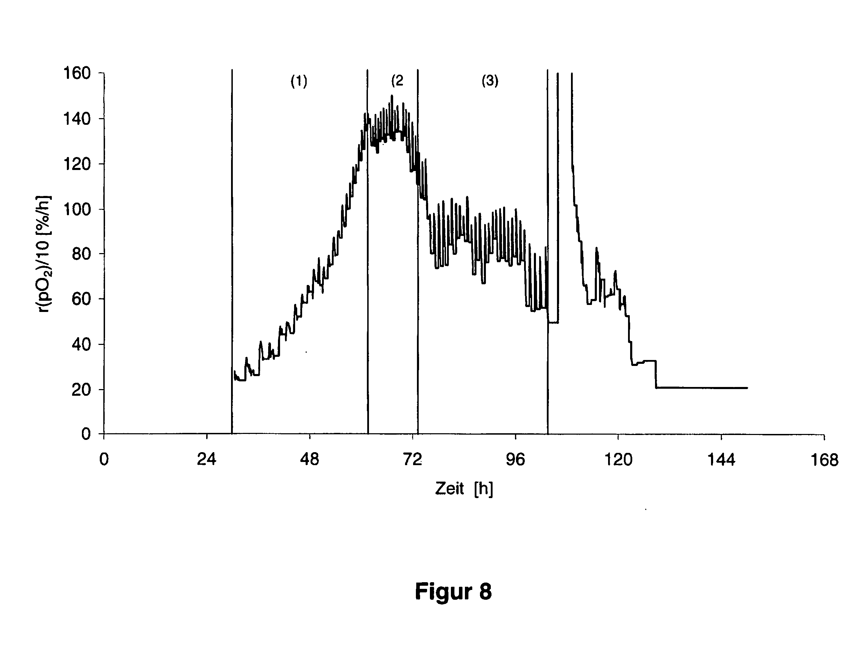

Die Sauerstoffverbrauchsrate (Figur 8) stieg in den ersten Tagen an, bis sie ein Maximum bei cirka 60 Stunden erreichte und nach 72 Stunden auf ein niedrigeres Plateau abfiel. Es waren eine exponentielle (1), eine verzögerte exponentielle (2) und eine stationäre (3) Wachstumsphase erkennbar. Figur 7 zeigt den Verlauf der Metabolite im Versuchsansatz A über eine Kultivierungsdauer von 7 Tagen (168 Stunden).

Im parallelen Versuchsansatz A (erfindungsgemäß) wurde die Kultur ebenfalls nach 4 Tagen mit der Standardmenge Influenzavirus und Trypsin infiziert. Gegenüber dem Kontrollansatz B wurde im Versuchsansatz A (erfindungsgemäß) weniger Glucose verbraucht. Am Ende der Zellanzucht waren noch 7,5 mmol/l Glucose im Medium. Die Lactatbildung war dementsprechend reduziert. Zum Infektionszeitpunkt waren in der Kultur cirka 23 mmol/l Lactat Angesammelt, demgegenüber waren im Kontrollansatz B bereits 37 mmol/l Lactat angesammelt. Die Lactatkonzentration war im erfindungsgemäß verwendeten Medium also fast 40 % geringer als im Ansatz mit Standard-GMEM. Die Lactatausbeute pro Glucose wurde beim Versuchsansatz A mit 88,5 % bestimmt und war damit gleich wie beim Kontrollansatz B.

Die Ammoniumkonzentration war während der gesamten Kultivierung sehr niedrig. Von anfänglich 0,44 mol/l sank die Konzentration unter 0,01 mol/l zum Zeitpunkt der Infektion. Zur gleichen Zeit waren in dem Kontrollansatz B (siehe oben) bereits cirka 2 mmol/l Ammonium angesammelt. Aus dem Verlauf der Ammoniumkonzentration über den Versuchszeitraum wird erkennbar, dass die kultivierten Zellen im Versuchsansatz A offensichtlich freie Ammoniumionen aus dem Medium aufnahmen.

Der Pyruvatverbrauch in der Wachstumsphase und auch in der Infektionsphase dokumentiert die hohe Wichtigkeit von Pyruvat als Kohlenstoff- und Energiequelle für die Zellen. Die in Figur 7 dargestellten Ergebnisse stehen im Einklang mit den Ergebnissen aus Beispiel 3.

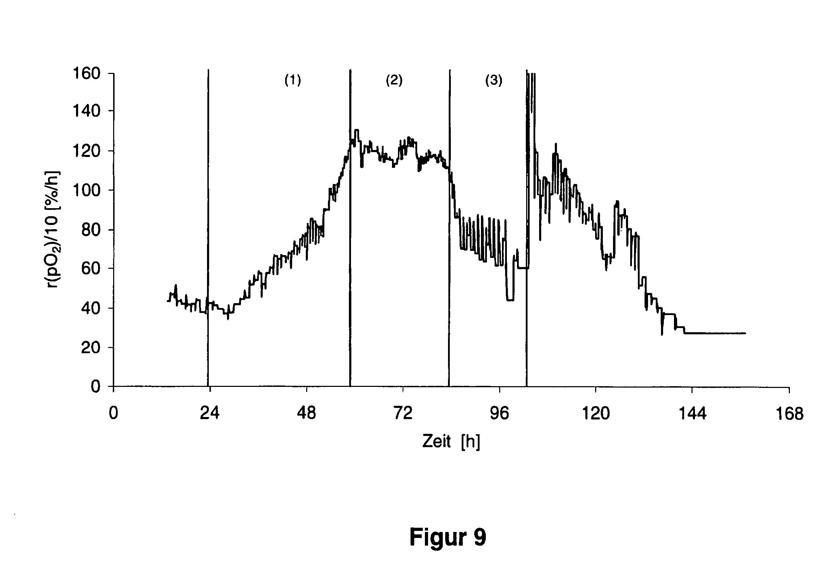

Die Sauerstoffverbrauchsrate (Figur 9) zeigte im Versuchsansatz A in den ersten zwei Tagen einen starken Anstieg (exponentielle Phase (1)), worauf eine Plateauphase (2) mit konstanter Sauerstoffverbrauchsrate folgte. Die Dauer der Plateauphase nach dem exponentiellen Anstieg war im Versuchsansatz A deutlich länger als in dem Kontrollansatz B. Im Versuchsansatz A dauerte die Plateauphase vom Zeitpunkt 59 Stunden nach Beginn der Kultivierung bis zum Zeitpunkt 81 Stunden nach Beginn der Kultivierung; demgegenüber begann im Kontrollansatz B die Plateauphase nach 61 Stunden und dauerte bis zum Zeitpunkt 71 Stunden. Am vierten Kulturtag sank die Sauerstoffverbrauchsrate innerhalb weniger Stunden auf ein zweites, niedrigeres Plateau stationärer Wachstumsphase (3). Der gesamte Sauerstoffverbrauch, erkennbar am Integral der r(pO$_2$)-Kurve, war im Versuchsansatz A damit größer als im Kontrollansatz B.

Die Infektion mit Viren fand in dieser Phase statt. Die stationäre Phase war im Versuchsansatz A um 16 Stunden gegenüber dem Kontrollansatz B verkürzt. Nach der Infektion war die Sauerstoffverbrauchsrate im Versuchsansatz A höher als im Kontrollansatz B. Die Sauerstoffaufnahme hielt fast 20 Stunden länger an als in dem Kontrollansatz, was einer längeren Virusproduktionsphase entspricht.

Die über den Hämagglutinationstest bestimmte Virenproduktion der beiden Ansätze zeigte bei der Kultur im Versuchsansatz A (erfindungsgemäß) eine virusbedingte Agglutination der Erythrozyten bis zu einem Verdünnungsfaktor zwischen 1:64 und 1:128 (log$_{10}$ (HA-Units) pro 100 $\mu$l = 1,95); demgegenüber betrug der Verdünnungsfaktor für die erfolgreiche Agglutination mit Viren aus dem Kontrollansatz B zwischen 1:32, und 1:64 (log$_{10}$ (HA-Units) pro 100 $\mu$l = 1,65). Das heißt, die Agglutination fand erst bei einer durchschnittlich doppelt so hohen Konzentration der Virenprobe statt, was auf eine deutlich geringere Virenproduktion im Kontrollansatz B im Vergleich zum Versuchsansatz A (erfindungsgemäß) hinweist.

*Konfluente Überwachung der Microcarrier*

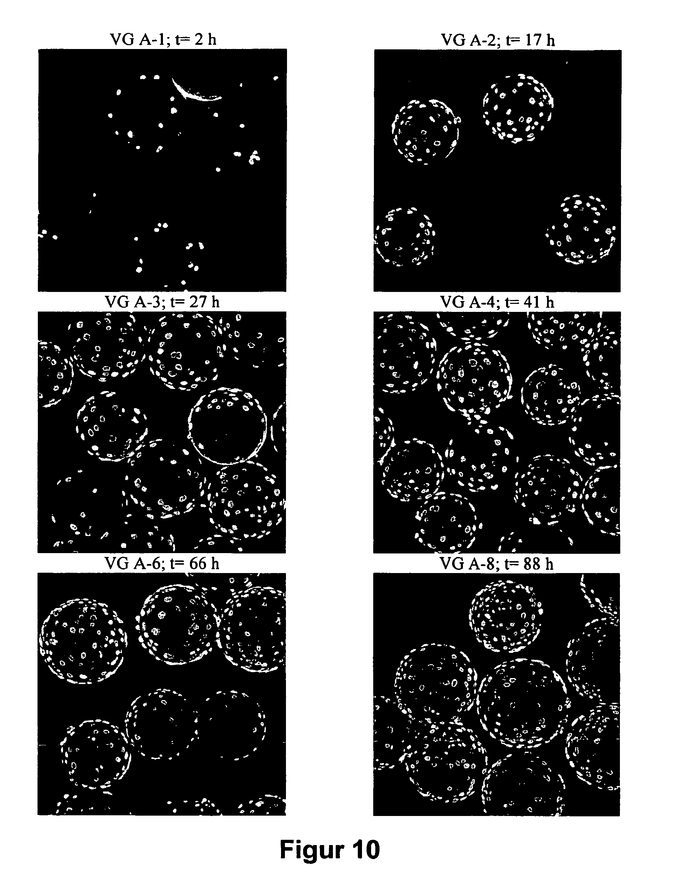

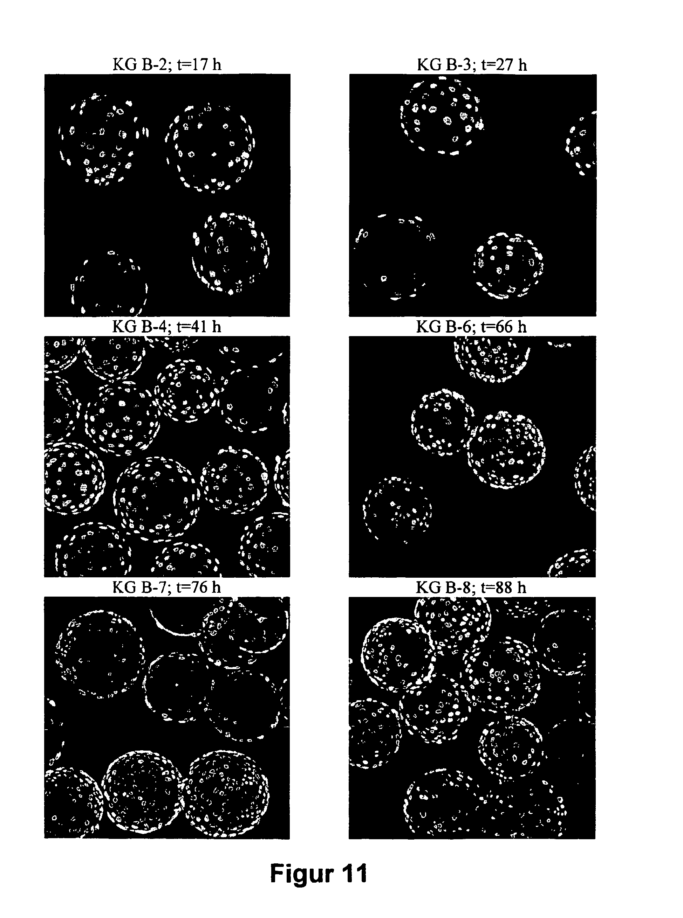

Figur 10 und Figur 11 zeigen mikroskopische Aufnahmen der Microcarrier aus Proben der Kontrollgruppe B und der Versuchsgruppe A zu verschiedenen Zeitpunkten. Sowohl in der Kontrollgruppe B wie auch in der Versuchsgruppe A waren die Carrier nach. 88 Stunden Kulturdauer vollständig konfluent bewachsen.

Beispiel 6: **Kultivierung von CHO-K1, BHK-21 und MDCK-Zellen in Glutamin-defizientem Medium**

a) Kultivierung und Medien

Es wurden jeweils Zellkulturen der Zelllinien MDCK (Beispiele 2 bis 5), CHO Typ K1 und BHK Typ 21 angelegt. Die Kultivierungsbedingungen entsprechen den in Beispiel 4 genannten Bedingungen, wobei Zellkulturflachen vom Typ T25 mit 25 cm$^2$ eingesetzt wurden.

Neben dem vorgenanntem Standardmedium GMEM wurden HAM's F12 und Optipro™SFM (Invitrogen) als weitere Standardmedien eingesetzt: Für die erfindungsgemäß verwendeten Medien wurde in den kommerziell erhältlichen glutaminfreien Versionen dieser Standardmedien 10 mmol/l Na-Pyruvat (Pyr) (entsprechend 1100 mg/l) supplementiert. In den Kontrollmedien war Glutamin in der in den Standardrezepturen angegebenen Konzentration enthalten.

b) Ergebnisse

Die Ergebnisse sind in Tabelle 2 dargestellt. Die Bestimmung der Medienparameter und der extrazellulären Metaboliten erfolgte gemäß Beispiel 3 c).

Die Kultivierung der Zellen in den erfindungsgemäß verwendeten glutaminfreien pyruvathaltigen Medien führte zu deutlich verminderter Ammoniumkontamination. Ebenfalls konnte eine verminderte Lactatbildung in festgestellt werden.

Tabelle 2:

| Zelllinie | Medium | Glutamin Verbrauch | NH$_4^+$ Bildung | Glucose Verbrauch | Lactat Bildung |
|---|---|---|---|---|---|
| CHO-K1 | GMEM | 1,1 | 1,9 | 13,8 | 25 |
| | GMEM (* | 0,06 | 0,17 | 10,5 | 21 |
| | Ham's F12 (* | 0,01 | 0,03 | 3 | 5,3 |
| BHK-21 | GMEM | 1,3 | 1,2 | 12,6 | 23,3 |
| | GMEM (* | 0,1 | 0,18 | 8,1 | 16,6 |

(fortgesetzt)

| Zelllinie | Medium | Glutamin Verbrauch | NH$_4^+$ Bildung | Glucose Verbrauch | Lactat Bildung |
|---|---|---|---|---|---|
| MDCK | Optipro SFM | 3,15 | 1,82 | 12 | 21,1 |
| | Optipro SFM (* | 0 | 0 | 8,1 | 19,8 |
| *) erfindungsgemäß | | | | | |

## Patentansprüche

1. Verwendung eines Zellkulturmediums zur Verminderung der Bildung von Lactat, Ammoniak und/oder Ammonium in einer Säugetierzellkultur, **dadurch gekennzeichnet, dass** der Anteil an Glutamin und/oder glutaminhaltigen Stoffen im Zellkulturmedium weniger als 0,2 mmol/l beträgt und gleichzeitig Pyruvat und/oder mindestens eine pyruvathaltige Verbindung in einer Konzentration von 2,5 bis 100 mmol/l im Zellkulturmedium vorhanden ist.

2. Verwendung eines Zellkulturmediums zur wachstumsfördernden Kultivierung von Säugetierzellen, **dadurch gekennzeichnet, dass** der Anteil an Glutamin und/oder glutaminhaltigen Stoffen im Zellkulturmedium weniger als 0,2 mmol/l beträgt und gleichzeitig Pyruvat und/oder mindestens eine pyruvathaltige Verbindung in einer Konzentration von 2,5 bis 100 mmol/l im Zellkulturmedium vorhanden ist.

3. Verwendung eines Zellkulturmediums nach einem der Ansprüche 1 oder 2, wobei Pyruvat im Zellkulturmedium in einer Konzentration von 5 bis 60 mmol/l vorhanden ist.

4. Verwendung eines Zellkulturmediums nach einem der Ansprüche 1 oder 2, wobei Pyruvat im Zellkulturmedium in einer Konzentration von 5 bis 20 mmol/l vorhanden ist.

5. Verwendung eines Zellkulturmediums nach einem der Ansprüche 1 bis 4, wobei Pyruvat im Zellkulturmedium als Natriumsalz und/oder als freie Säure vorhanden ist.

6. Verwendung eines Zellkulturmediums nach einem der Ansprüche 1 bis 5, wobei das Zellkulturmedium frei von Glutamin und glutaminhaltigen Stoffen ist.

7. Verfahren zur Kultivierung von Säugetierzellen, **dadurch gekennzeichnet, dass** ein Zellkulturmedium eingesetzt wird, wobei der Anteil an Glutamin und/oder glutaminhaltigen Stoffen im Zellkulturmedium weniger als 0,2 mmol/l beträgt und gleichzeitig Pyruvat und/oder mindestens eine pyruvathaltige Verbindung in einer Konzentration von 2,5 bis 100 mmol/l im Zellkulturmedium vorhanden ist.

8. Verfahren nach Anspruch 7, wobei ein in den Ansprüchen 3 bis 6 genanntes Zellkulturmedium verwendet wird.

## Claims

1. Use of a cell culture medium to reduce the formation of lactate, ammonia and/or ammonium in a mammalian cell culture, **characterized in that** the content of glutamine and/or glutamine-containing substances in the cell culture medium is less than 0.2 mmol/l and at the same time pyruvate and/or at least one pyruvate-containing compound is present in a concentration of from 2.5 to 100 mmol/l in the cell culture medium.

2. Use of a cell culture medium for the growth-promoting cultivation of mammalian cells, **characterized in that** the content of glutamine and/or glutamine-containing substances in the cell culture medium is less than 0.2 mmol/l and at the same time pyruvate and/or at least one pyruvate-containing compound is present in a concentration of from 2.5 to 100 mmol/l in the cell culture medium.

3. Use of a cell culture medium according to one of claims 1 or 2, wherein pyruvate is present in the cell culture medium in a concentration of from 5 to 60 mmol/l.

4. Use of a cell culture medium according to one of claims 1 or 2, wherein pyruvate is present in the cell culture medium in a concentration of from 5 to 20 mmol/l.

**5.** Use of a cell culture medium according to one of claims 1 to 4, wherein pyruvate is present in the cell culture medium as sodium salt and/or as a free acid.

**6.** Use of a cell culture medium according to one of claims 1 to 5, wherein the cell culture medium is free of glutamine and glutamine-containing substances.

**7.** Method for cultivating mammalian cells, **characterized in that** a cell culture medium is used, the content of glutamine and/or glutamine-containing substances in the cell culture medium being less than 0.2 mmol/l and at the same time pyruvate and/or at least one pyruvate-containing compound being present in a concentration of from 2.5 to 100 mmol/l in the cell culture medium.

**8.** Method according to claim 7, wherein a cell culture medium mentioned in claims 3 to 6 is used.


**Revendications**

**1.** Utilisation d'un milieu de culture cellulaire pour réduire la formation de lactate, d'ammoniac et/ou d'ammonium dans une culture de cellules de mammifère, **caractérisée en ce que** la proportion de glutamine et/ou de substances contenant de la glutamine dans le milieu de culture cellulaire est inférieure à 0,2 mmol/l et qu'en même temps du pyruvate et/ou au moins un composé contenant du pyruvate est disponible en une concentration de 2,5 à 100 mmol/l dans le milieu de culture cellulaire.

**2.** Utilisation d'un milieu de culture cellulaire pour cultiver des cellules de mammifère de manière à favoriser leur croissance, **caractérisée en ce que** la proportion de glutamine et/ou de substances contenant de la glutamine dans le milieu de culture cellulaire est inférieure à 0,2 mmol/l et qu'en même temps du pyruvate et/ou au moins un composé contenant du pyruvate est disponible en une concentration de 2,5 à 100 mmol/l dans le milieu de culture cellulaire.

**3.** Utilisation d'un milieu de culture cellulaire selon l'une des revendications 1 ou 2, le pyruvate étant disponible dans le milieu de culture cellulaire en une concentration de 5 à 60 mmol/l.

**4.** Utilisation d'un milieu de culture cellulaire selon l'une des revendications 1 ou 2, le pyruvate étant disponible dans le milieu de culture cellulaire en une concentration de 5 à 20 mmol/l.

**5.** Utilisation d'un milieu de culture cellulaire selon l'une des revendications 1 à 4, le pyruvate étant disponible dans le milieu de culture cellulaire en tant que sel de sodium et/ou d'acide libre.

**6.** Utilisation d'un milieu de culture cellulaire selon l'une des revendications 1 à 5, le milieu de culture cellulaire étant dépourvu de glutamine et de substances contenant de la glutamine.

**7.** Procédé de culture de cellules de mammifère, **caractérisé en ce qu'**un milieu de culture cellulaire est utilisé, la proportion de glutamine et/ou de substances contenant de la glutamine dans le milieu de culture cellulaire étant inférieure à 0,2 mmol/l et du pyruvate et/ou au moins un composé contenant du pyruvate étant en même temps disponible en une concentration de 2,5 à 100 mmol/l dans le milieu de culture cellulaire.

**8.** Procédé selon la revendication 7, un milieu de culture cellulaire cité dans les revendications 3 à 6 étant utilisé.

| Medium a)<br>GMEM ohne Glutamin | Medium b)<br>Glycerin 9,8 mmol/lol/l | Medium c)<br>Citrat 1,24 mmol/lol/l | |
|---|---|---|---|
| Medium d)<br>Pyruvat 2,5 mmol/l | Medium e)<br>Pyruvat 5 mmol/l | Medium f)<br>Pyruvat 9,8 mmol/l | Medium g)<br>Pyruvat 19,2 mmol/l |
| Medium h)<br>Pyruvat 5 mmol/l<br>Glycerin 5 mmol/l | Medium i)<br>Citrat 2,5 mmol/l<br>Glycerin 5 mmol/l | Medium k)<br>Pyruvat 5 mmol/l<br>Citrat 2,5 mmol/l | Medium l)<br>Pyruvat 5 mmol/l<br>Citrat 2,5 mmol/l<br>Glycerin 5 mmol/l |
| Medium m)<br>Pyruvat 5 mmol/l<br>Glycerin 5 mmol/l<br>Glutamin 4 mmol/l | Medium n)<br>Citrat 2,5 mmol/l<br>Glycerin 5 mmol/l<br>Glutamin 4 mmol/l | Medium o)<br>Pyruvat 5 mmol/l<br>Citrat 2,5 mmol/l<br>Glutamin 4 mmol/l | Medium p)<br>Pyruvat 5 mmol/l<br>Citrat 2,5 mmol/l<br>Glycerin 5 mmol/l<br>Glutamin 4 mmol/l, |

**Figur 1**

Figur 2

Zellwachstum in Spinnerflaschen

Figur 3

**Figur 4**

Spinnerflasche mit Medium I (erfindungsgemäß)

Figur 5

**Figur 6**

Figur 7

**Figur 8**

EP 1 516 045 B1

Figur 9

VG A-1; t= 2 h

VG A-2; t= 17 h

VG A-3; t= 27 h

VG A-4; t= 41 h

VG A-6; t= 66 h

VG A-8; t= 88 h

**Figur 10**

KG B-2; t=17 h

KG B-3; t=27 h

KG B-4; t=41 h

KG B-6; t=66 h

KG B-7; t=76 h

KG B-8; t=88 h

**Figur 11**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **FITZPATRICK et al.** *Appl. Biochem. Biotechnol.,* 1993, vol. 43 (2), 93-116
- **NEERMANN ; WAGNER.** *J. Cell. Physiol.,* 1996, vol. 166, 152-169
- **GLACKEN.** *Bio/Technology,* 1988, vol. 6, 1941-1950
- **REITZER et al.** *J. Biol. Chem.,* 1979, vol. 254 (8), 2669-2676
- **OZTURK.** Encyclopedia of Cell Technology. John Wiley & Sons, 2000, 121-136
- **SCHNEIDER et al.** *J. Biotechnol.,* 1996, vol. 46, 161-185
- **KURANO et al.** *J. Biotechnol.,* 1990, vol. 15, 113-128
- **MC-DERMOTT ; BUTLER.** *J. Cell. Sci.,* 1993, vol. 104, 51-58
- **ALTAMIRANO et al.** *Animal Cell Technology: Products from Cells, Cells as Products,* 1999, 95-97
- **NADEAU et al.** *Met. Eng.,* 2000, vol. 2, 277-292
- **DOVERSKOG et al.** *J. Biotechnol.,* 1997, vol. 59, 103-115
- **DOWNS ; HUDSON.** *Zyqote,* 2000, vol. 8, 339-351
- **VON EAGLE.** *Science,* 1955, vol. 122, 501
- *Science,* 1959, vol. 130, 432-437
- **DULBECCO.** *Virology,* 1959, vol. 8, 396-397
- **GLACKEN et al.** *Biotechnol. Bioeng.,* 1986, vol. 32, 1015-1028